# EUROPEAN PATENT SPECIFICATION

(11) **EP 2 015 775 B1**
(45) Date of publication and mention of the grant of the patent: **01.06.2011**
(21) Application number: 07724950.6
(22) Date of filing: 26.04.2007
(51) Int. Cl.: A61K 39/395, A61P 35/00, C07K 16/28, C07K 16/18, A61K 51/10

(54) **COMBINATION OF AN ANTI EDB FIBRONECTIN DOMAIN ANTIBODY L19-SIP AND AN ANTI-EGFR ANTIBODY**
KOMBINATION AUS EINEM ANTI-EDB-FIBRONEKTIN-DOMÄNEN-ANTIKÖRPER L19-SIP UND EINEM ANTI-EGFR-ANTIKÖRPER
COMBINAISON D'UN ANTICORPS L19-SIP ANTI DOMAINE ED-B DE LA FIBRONECTINE ET D'UN ANTICORPS ANTI-EGFR

(30) Priority: 03.05.2006 EP 06090067; 15.05.2006 US 800011 P
(43) Date of publication of application: 21.01.2009
(73) Proprietor: Bayer Schering Pharma Aktiengesellschaft, 13353 Berlin (DE); Vereniging VU-Windesheim, 1081 HV Amsterdam (NL)
(72) Inventor: SIEGER, Stephanie, 74930 Ittlingen (DE); BERNDORFF, Dietmar, 13467 Berlin (DE); DINKELBORG, Ludger, 13465 Berlin (DE); TIJINK, Bernhard, Marcel, NL-1065 DB Amsterdam (NL); VAN DONGEN, Augustinus, Antonius, Maria, Silvester, NL-3572 HL Utrecht (NL)
(86) International application number: PCT/EP2007/004026
(87) International publication number: WO 2007/128557

(56) References cited:
- WO-A-03/055917
- WO-A-2003/076469
- D. BERNDORFF ET AL.: "Radioimmunotherapy of solid tumors by targeting extra domain B fibronectin: Identification of the best-suited radioimmunoconjugate." CLINICAL CANCER RESEARCH, vol. 11, no. 19(suppl.), 1 October 2005 (2005-10-01), pages 7053S-7063S, XP002405040 cited in the application
- F. VAN GOG ET AL.: "Perspectives of combined radioimmunotherapy and anti-EGFR antibody therapy for the treatment of residual head and neck cancer." INTERNATIONAL JOURNAL OF CANCER, vol. 77, 1998, pages 13-18, XP002405039 cited in the application
- H. VAN CRUIJSEN ET AL.: "Epidermal growth factor receptor and angiogenesis: opportunities for combined anticancer strategies." INTERNATIONAL JOURNAL OF CANCER, vol. 117, no. 6, 20 December 2005 (2005-12-20), pages 883-888, XP002405041 cited in the application
- F. VITI ET AL.: "Increased binding affinity and valence of recombinant antibody fragments lead to improved targeting of tumoral angiogenesis." CANCER RESEARCH, vol. 59, 15 January 1999 (1999-01-15), pages 347-352, XP002124782 cited in the application
- L. BORSI ET AL.: "Selective targeting of tumoral vasculature: Comparison of different formats of an antibody (L19) to the ED-B domain of fibronectin." INTERNATIONAL JOURNAL OF CANCER, vol. 102, 1 November 2002 (2002-11-01), pages 75-85, XP002252051
- B. TIJINK ET AL.: "Radioimmunotherapy of head and neck cancer xenografts using 131I-labeled antibody L19-SIP for selective targeting of tumor vasculature." JOURNAL OF NUCLEAR MEDICINE, vol. 47, no. 7, July 2006 (2006-07), pages 1127-1135, XP002405042 USA

### SEQUENCE LISTING

<110> Bayer Schering Pharma Aktiengesellschaft
<120> Combination of an anti ED-B fibronectin domain antibody and an anti-EGFR antibody
<130> 53421AWO
<150> EP06090067.7
   <151> 2006-05-03
   <150> US60/800011
   <151> 2006-05-15
<160> 13
<170> PatentIn version 3.1
<210> 1
   <211> 116
   <212> PRT
   <213> Artificial
<220>
   <221> DOMAIN
   <222> (1)..(116)
   <223> Antibody-VH-Region from L19
<400> 1
<210> 2
   <211> 108
   <212> PRT
   <213> Artificial
<220>
   <221> DOMAIN
   <222> (1)..(108)
   <223> Antibody-VL-Region from L19
<400> 2
<210> 3
   <211> 12
   <212> PRT
   <213> Artificial
<220>
   <221> DOMAIN
   <222> (1)..(12)
   <223> Antibody linker region
<400> 3
   Gly Asp Gly Ser Ser Gly Gly Ser Gly Gly Ala Ser
   1 5 10
<210> 4
   <211> 116
   <212> PRT
   <213> Artificial
<220>
   <221> DOMAIN
   <222> (1)..(116)
   <223> etas2-CH4 domain
<400> 4
   Gly Pro Arg Ala Ala Pro Glu Val Tyr Ala Phe Ala Thr Pro Glu Trp
   1 5 10 15
   Pro Gly Ser Arg Asp Lys Arg Thr Leu Ala Cys Leu Ile Gln Asn Phe
   20 25 30
   Met Pro Glu Asp Ile Ser Val Gln Trp Leu His Asn Glu Val Gln Leu
   35 40 45
   Pro Asp Ala Arg His Ser Thr Thr Gln Pro Arg Lys Thr Lys Gly Ser
   50 55 60
   Gly Phe Phe Val Phe Ser Arg Leu Glu Val Thr Arg Ala Glu Trp Glu
   65 70 75 80
   Gln Lys Asp Glu Phe Ile Cys Arg Ala Val His Glu Ala Ala Ser Pro
   85 90 95
   Ser Gln Thr Val Gln Arg Ala Val Ser Val Asn Pro Glu Ser Ser Arg
   100 105 110
   Arg Gly Gly Cys
   115
<210> 5
   <211> 5
   <212> PRT
   <213> Artificial
<220>
   <221> DOMAIN
   <222> (1)..(5)
   <223> fusion protein linker
<400> 5
   Ser Gly Gly Ser Gly
   1 5
<210> 6
   <211> 5
   <212> PRT
   <213> Artificial
<220>
   <221> DOMAIN
   <222> (1)..(5)
   <223> CUR1 of VH L19
<400> 6
   Ser Phe Ser Met Ser
   1 5
<210> 7
   <211> 17
   <212> PRT
   <213> Artificial
<220>
   <221> DOMAIN
   <222> (1)..(17)
   <223> CDR2 of VH L19
<400> 7
   Ser Ile Ser Gly Ser Ser Gly Thr Thr Tyr Tyr Ala Asp Ser Val Lys
   1 5 10 15
   Gly
<210> 8
   <211> 7
   <212> PRT
   <213> Artificial
<220>
   <221> DOMAIN
   <222> (1)..(7)
   <223> CDR3 of VH L19
<400> 8
   Pro Phe Pro Tyr Phe Asp Tyr
   1 5
<210> 9
   <211> 12
   <212> PRT
   <213> Artificial
<220>
   <221> DOMAIN
   <222> (1)..(12)
   <223> CDR1 of vl L19
<400> 9
   Arg Ala Ser Gln Ser Val Ser Ser Ser Phe Leu Ala
   1 5 10
<210> 10
   <211> 7
   <212> PRT
   <213> Artificial
<220>
   <221> DOMAIN
   <222> (1)..(7)
   <223> CDR2 of vl L19
<400> 10
   Tyr Ala Ser Ser Arg Ala Thr
   1 5
<210> 11
   <211> 9
   <212> PRT
   <213> Artificial
<220>
   <221> DOMAIN
   <222> (1)..(9)
   <223> CDR3 of vl L19
<400> 11
   Gln Gln Thr Gly Arg Ile Pro Pro Thr
   1 5
<210> 12
   <211> 357
   <212> PRT
   <213> Artificial
<220>
   <221> DOMAIN
   <222> (1)..(357)
   <223> sequence of L19-SIP in its monomeric form
<400> 12
   Glu Val Gln Leu Leu Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Phe
               20                  25                  30
   Ser Met Ser Trp Val Arg Gln Ala Pro Gly Lys Gly Leu Glu Trp Val
           35                  40                  45
   Ser Ser Ile Ser Gly Ser Ser Gly Thr Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Lys Pro Phe Pro Tyr Phe Asp Tyr Trp Gly Gln Gly Thr Leu Val
               100                 105                 110
   Thr Val Ser Ser Gly Asp Gly Ser Ser Gly Gly Ser Gly Gly Ala Ser
           115                 120                 125
   Glu Ile Val Leu Thr Gln Ser Pro Gly Thr Leu Ser Leu Ser Pro Gly
       130                 135                 140
   Glu Arg Ala Thr Leu Ser Cys Arg Ala Ser Gln Ser Val Ser Ser Ser
   145                 150                 155                 160
   Phe Leu Ala Trp Tyr Gln Gln Lys Pro Gly Gln Ala Pro Arg Leu Leu
                   165                 170                 175
   Ile Tyr Tyr Ala Ser Ser Arg Ala Thr Gly Ile Pro Asp Arg Phe Ser
   180 185 190
   Gly Ser Gly Ser Gly Thr Asp Phe Thr Leu Thr Ile Ser Arg Leu Glu
   195 200 205
   Pro Glu Asp Phe Ala Val Tyr Tyr Cys Gln Gln Thr Gly Arg Ile Pro
   210 215 220
   Pro Thr Phe Gly Gln Gly Thr Lys Val Glu Ile Lys Ser Gly Gly Ser
   225 230 235 240
   Gly Gly Pro Arg Ala Ala Pro Glu Val Tyr Ala Phe Ala Thr Pro Glu
   245 250 255
   Trp Pro Gly Ser Arg Asp Lys Arg Thr Leu Ala Cys Leu Ile Gln Asn
   260 265 270
   Phe Met Pro Glu Asp Ile Ser Val Gln Trp Leu His Asn Glu Val Gln
   275 280 285
   Leu Pro Asp Ala Arg His Ser Thr Thr Gln Pro Arg Lys Thr Lys Gly
   290 295 300
   Ser Gly Phe Phe Val Phe Ser Arg Leu Glu Val Thr Arg Ala Glu Trp
   305 310 315 320
   Glu Gln Lys Asp Glu Phe Ile Cys Arg Ala Val His Glu Ala Ala Ser
   325 330 335
   Pro Ser Gln Thr Val Gln Arg Ala Val Ser Val Asn Pro Glu Ser Ser
   340 345 350
   Arg Arg Gly Gly Cys
   355
<210> 13
   <211> 17
   <212> PRT
   <213> Artificial
<220>
   <221> DOMAIN
   <222> (1)..(17)
   <223> alternative antibody or fusion protein linker
<400> 13
   Glu Phe Ser Ser Ser Ser Gly Ser Ser Ser Ser Gly Ser Ser Ser Ser
   1 5 10 15
   Gly

## Description

The invention relates to a combination (i) of a fusion protein, comprising an εₛ₂-CH4 domain part and an antibody part, specifically recognising the extra domain B of fibronectin (ED-B - fibronectin), and wherein the fusion protein is in dimeric form and wherein the fusion protein is conjugated to a radioisotope and (ii) and an anti-EGFR-antibody and its use for treatment of cancer, in particular head and neck cancer, non-small cell lung cancer, prostate cancer, colorectal cancer, ovarian cancer, pancreatic cancer, gastric cancer, and / or breast cancer.

### STATE OF THE ART

One of the most selective oncofetal markers associated with neo-angiogenesis and tissue remodeling known so far represents the extra domain B (ED-B) of Fibronectin (FN) (Castellani P, Viale G, Dorcaratto A, Nicolo G, Kaczmarek J, Querze G, Zardi L. The fibronectin isoform containing the ED-B oncofetal domain: a marker of angiogenesis. Int J Cancer. 1994 Dec 1;59(5):612-8. Erratum in: Int J Cancer 1995 Jul 4;62(1):118.. FNs are high molecular-weight extracellular matrix (ECM) components abundantly expressed in a range of healthy tissues and body fluids. Various different FN isoforms can be generated due to alternative splicing at the level of the primary transcript. The ED-B, a small domain of 91 amino acids, which is identical in sequence in mouse and man, is usually absent in both plasma and tissue-fibronectin, except for some blood vessels of the regenerating endometrium and the ovaries. (Alessi P, Ebbinghaus C, Neri D. Molecular targeting of angiogenesis. Biochim Biophys Acta 2004;1654:39-49; Viti F, Tarli L, Giovannoni L, Zardi L, Neri D. Increased binding affinity and valence of recombinant antibody fragments lead to improved targeting of tumoral angiogenesis. Cancer Res 1999;59:347-352). However, it may become inserted in the fibronectin molecule during active tissue remodeling associated with neo-angiogenesis, thereby accumulating around the neo-vasculature and in the stroma of malignant tumors and in other tissues undergoing remodeling and angiogenesis. Recently, a,number of good quality antibodies specific for the ED-B domain of fibronectin have been generated. In particular, the human single chain Fv antibody fragment scFv(L19), which displays a picomolar binding affinity for ED-B, has been verified to selectively target tumor neovasculature, both in experimental tumor models (Viti F, Tarli L, Giovannoni L, Zardi L, Neri D. Increased binding affinity and valence of recombinant antibody fragments lead to improved targeting of tumoral angiogenesis. Cancer Res 1999;59:347-352.) and in patients with cancer (Santimaria M, Moscatelli G, Viale GL, Giovannoni L, Neri G, Viti F, Leprini A, Borsi L, Castellani P, Zardi L, Neri D, Riva P. Immunoscintigraphic detection of the ED-B domain of fibronectin, a marker of angiogenesis, in patients with cancer. Clin Cancer Res 2003;9:571-579.).

The ED-B domain of fibronectin, a sequence of 91 amino acids identical in mice, rats and humans, which is inserted by alternative splicing into the fibronectin molecule, specifically accumulates around neovascular structures and represents a target for molecular intervention (Zardi et al. (1987) EMBO J. Vol.: 6, pages 2337 - 2342 ; Carnemolla et al. (1989) J. Cell Biol. Vol: 108, pages 1139 - 1148, further Castellani et al, (1994) Int. J. Cancer, Vol. 59, pages 612 - 618). Using the human recombinant antibody L19 directed to the ED-B domain, the possibility of in vivo neovasculature targeting has been demonstrated in different tumour models (Tarli et al. (1999) Blood, Vol.: 94, pages 192 - 198; Viti et al. (1999) Cancer Res. Vol.: 347).

Monoclonal antibodies specifically recognising the ED-B - fibronectin domain are described in WO 97/45544.

Monoclonal antibody L19 is described in WO 99/58570.

WO 01/62298 on page 8, line 12 refers to the L19 VH and L19 VL domain sequences described in Pini et al. (1998) J. Biol. Chem. 273: 21769-21776. Pini et al. describes parts of the sequence of L19 in Table II on page 21772. L19 has the EMBL accession Number AJ 006113.

Tumours cannot grow beyond a certain mass without the formation of new blood vessels (angiogenesis), and a correlation between micro vessel density and tumour invasiveness has been reported for a number of tumours (Folkmann (1995) Nat. Med., Vol. 1, page 27). Molecules capable of selectively targeting markers of angiogenesis create clinical opportunities for the diagnosis and therapy of tumours and other diseases characterised by vascular proliferation, such as rheumatoid arthritis, diabetic retinopathy and age-related macular degeneration (O'Reilly et al. (1996) Nat. Med. Vol.: 2 page 689, further O'Reilly et al. (1997) Cell, Vol.: 88, page 277, further Friedlander et al. (1995) Science Vol.: 270, page 1500, further Pasqualini et al. (1997) Nat. Biotechnol. Vol.: 15 page 542, further Huang et al. (1997) Science, Vol.: 275, page 547, further Kim et al. (1993) Nature, Vol.: 362, page 841, further Schmidt - Erfurth et al. (1997) Br. J. Cancer, Vol.: 75, page 54).

The principles of radiotargeting for cancer therapy are described by Kassis AI (2005) in Expert Opin. Drug Delivery Vol.: 2(6), 981-991 and by Press OW et al (2000) in Seminars in Oncology Vol.:27, No.6 (Suppl. 12), 62-73.

L19-SIP and its use for radioimmunotherapy are described in WO 03/076469. Berndorff et al (2005) Clin. Cancer Res. Vol.:11 (19 Suppl.), 7053S-70635 described the use of ¹³¹ I-L19-SIP as preferred antibody format for radioimmunotherapy of solid tumours by targeting the extra domain B fibronectin. "SIP" stands for small immunoprotein. L19-SIP is an antibody format wherein the L 19-scFv is linked to an εs2-CH4 domain, and wherein two monomeric chains form a homodimer covalently linked by an S-S bridge (see e.g. WO03/076469; Borsi et al., 2002, Int. J. Cancer, 102: 28: 534-539). CH4 is the domain that allows dimerization in the IgE molecule and the εs2- isoform contains a cysteine at the carboxyterminal end, which stabilizes the IgE-dimer through an inter-chain disulphide bond. In the final SIP molecule of L19-SIP the ScFv(L19) is connected to the εs2-CH4 domain by a GGSG linker.

Anti EGF receptor antibodies:

The first step in the mitogenic stimulation of epithelial cells is the specific binding of epidermal growth factor (EGF) to a membrane glycoprotein known as the epidermal growth factor receptor (EGF receptor) Carpenter et al. (1979) Annual Review Biochem., Vol.: 48, pages 193 - 216. The EGF receptor is composed of 1186 amino acids which are divided into an extra - cellular portion of 621 residues and a cytoplasmic portion of 542 residues connected by a single hydrophobic trans - membrane segment of 23 residues described in Ulrich et al. (1986) Nature, Vol.: 309, pates 418 - 425. The external portion of the EGF receptor can be subdivided into four domains. The domain III,. residues 333 to 460, which is connected by two cysteine domains, contains the EGF binding site of the receptor shown by Lax et al. (1988) Mol. and Cell Biol. Vol.: 8 pages 1831 to 1834. The binding of EGF to domain III leads to the initiation of pleiotropic responses leading to DNA synthesis and cell proliferation.

Various types of human tumour cells show overexpression of the EGF receptor. For example, the cancerous cells of bladder tumours have been shown to have a relatively large population of EGF receptors described in Neal et al. (1985) Lancet, Vol.: 1 pages 366-367. The influence of EGF receptor density on the biological behaviour of cancer cells may be mediated by the interaction of the receptor with its ligands - namely, EGF or transforming growth factor-α (TGF-α). In the majority of cells, when EGF binds to a specific region of the EGF receptor, the cell is mitogenically simulated. Other tumour cells, such as A431 cells, are not mitogenically stimulated by the binding of EGF to its receptor. Nevertheless, the tumour A413 is inhibited in nude mice by binding monoclonal antibodies to the epidermal growth factor receptor of the tumorous cells as shown by Masui et al. (1984) Cancer Res. Vol.: 44, pages 1002 - 1007. EP 0 359 282 describes an antibody specifically binding and inhibiting the growth of human tumours cells.

In 1993 Naramura et al (1993) Cancer Immunol. Immunotherapy Vol.: 37, 343 - 349 describes the antibody Cetuximab or C225, which recognises and binds to the EGF receptor. The problem with antibodies to coagulate and to aggregate is solved in WO 2003 / 007988.

There is a strong medical need for a medicament to effectively treat cancer, in particular head and neck cancer, non-small cell lung cancer, prostate cancer, colorectal cancer, ovarian cancer, pancreatic cancer, gastric cancer, and / or breast cancer. The present invention makes available novel and effective medicaments, which are suitable for the treatment of cancer, in particular head and neck cancer, non-small cell lung cancer, prostate cancer, colorectal cancer, ovarian cancer, pancreatic cancer, gastric cancer, and / or breast cancer.

The present invention relates to a combination comprising at least a fusion protein and an anti-EGFR-antibody,
wherein the fusion protein in its monomeric form comprises an εₛ₂-CH4 domain part and
an antibody - part specifically recognising the ED-B- fibronectin domain, and wherein the fusion protein is present in dimeric form and
wherein the fusion protein is conjugated to a radioisotope.

In a preferred embodiment, the anti-EGFR antibody is selected from Cetuximab (Erbitux®, Panitumomab (Vectibix®), Zalutumomab (HuMax-EGFr), Matuzumab (EMD72000), and an anti-EGFRvIII antibody.

In an especially preferred embodiment of the invention, the anti-EGFR antibody is Cetuximab.

Cetuximab antibody or C225 is a genetically engineered chimeric murine - human monoclonal antibody directed against the EGF receptor antigen. Cetuximab is the antibody called described in Naramura et al. (1993) Cancer Immunol. Immunotherapy, Vol.: 37, pages 343 - 349. Cetuximab is marketed as Erbitux.

EGF (Epidermal Growth Factor) and EGF Receptor (EGFR) have been described and characterised for example in Irit LAX et al. (1988) "Localization of a Major Receptor - Binding Domain for Epidermal Growth Factor by Affinity Labeling" Molecular and Cellular Biology, Vol 8 No. 4, pages 1831 - 1834 and references therein; and Graham CARPENTER (1979) "Epidermal Growth Factor" Ann. Rev. Biochem. Vol.: 48, pages 193 - 216. The term "EGFR" or "EGF-Receptor" according to the present invention encompasses full length and native EGFR and truncated versions thereof, in particular EGFRvIII. In a preferred embodiment, the term "EGFR" or "EGF-Receptor" according to the present invention is to be understood as meaning human full length and native EGFR.

The antibody Cetuximab or C225 is described inter alia in EP 0 359 282. A formulation of Cetuximab is published in WO 03 / 007988.

Cetuximab is indicated (1) in combination with irinotecan in patients with EGFR-expressing colorectal cancer who have failed prior irinotecan therapy, (2) in combination with radiotherapy as first line treatment for locally advanced squamous cell carcinoma of the head and neck, and (3) in combination with cisplatin as first line treatment for recurrent of metastatic squamous cell carcinoma of the head and neck .

Synergy between Cetuximab and chemotherapies and toxins has also been observed experimentally.

It was surprisingly it was found that the combination comprising at least a fusion protein and an anti-EGFR-antibody, wherein the fusion protein in its monomeric form comprises an εₛ₂-CH4 domain part and an antibody - part specifically recognising the ED-B- fibronectin domain and wherein the fusion protein is present the dimeric form and wherein the fusion protein is conjugated to a radioisotope can be used much more successfully for treatment of cancer than either the fusion - protein alone or an anti-EGFR-antibody alone.

The fusion protein is present in dimeric form, wherein the monomers are linked by a S-S bridge, as described in Borsi et al., Int. J. Cancer, 2002, 102: 534-539.

In a preferred embodiment, the fusion protein in its monomeric form has aN - terminal antibody - part and C - terminal εₛ₂-CH4 domain part or
wherein the fusion protein in its monomeric form has a N - terminal εₛ₂-CH4 domain part and a C - terminal antibody - part.

Especially preferred is the embodiment, wherein the fusion protein in its monomeric form has a N - terminal antibody - part and C - terminal εₛ₂-CH4 domain part.

In another preferred embodiment, the fusion protein in its monomeric form comprises a linker connecting the antibody - part and εₛ₂-CH4 domain part.

Preferably, the antibody-part is human.

In a preferred embodiment, the antibody-part specifically recognising the ED-B domain of fibronectin binds with sub-nanomolar affinity. For a review on the definitions and measurements of anti body-antigen affinity, see Ned et al. (1996). Trends in Biotechnol. 14, 465-470.

In a preferred embodiment, the antibody-part contains at least one of the CDRs of L19 antibody, preferably it contains all CDR sequences of the L19 antibody.

In a preferred embodiment, the antibody-part comprises at least one of the SEQ ID. No. 6 to 11. Preferably the antibody comprises the sequences according to SEQ ID no. 6 to 11.

In another preferred embodiment, the antibody-part comprises at least one V heavy chain according to Seq. Id. No. 01 or at least one V light chain according to Seq. Id. No. 02. In a more preferred embodiment, the antibody part comprises at least one V heavy chain according to Seq. Id. No. 01 and at least one V light chain according to Seq. Id. No. 02. In an even more preferred embodiment, the antibody-part comprises one V heavy chain according to Seq. Id. No. 01 and one V light chain according to Seq. Id. No. 02.

Preferred is an antibody part, wherein the heavy and the light chain are connected by an antibody linker.

In a preferred embodiment ,the antibody linker comprises a sequence according to Seq. Id. No. 03, or a sequence having at least 90% identity to the sequence according to Seq. Id. No. 03. In a more preferred embodiment, the antibody linker has a sequence according to Seq. Id. No. 03..

In a more preferred embodiment, the eₛ₂-CH4 domain part comprises a sequence according to the Seq. Id No 04.

Preferred is the use of the fusion protein in its monomeric form according to the invention, wherein the fusion protein linker comprises a sequence according to the Seq. Id No 05, preferably the fusion protein linker has a sequence according to the Seq. Id No 05.

In a preferred embodiment the fusion protein linker has a length of 0 to 15 amino acids, more preferred 1 to 10, even more preferred 1 to 6 amino acids.

In the combination according to the invention, the fusion protein is conjugated to a radioisotope.. Methods for labelling fusion proteins of the invention are disclosed in Berndorff et al., Clin. Cancer Res., 2005; 11 (Suppl.), p. 7053s-7063s.

In a preferred embodiment, the radioisotope is a radioisotope selected from I; Tc, Re, In, Y, Lu, or I or a mixture thereof.

In an even more preferred embodiment, the radioisotope is selected from ¹²³I, ¹²⁴I,¹²⁵I, ¹³¹I, ^{99m}Tc, ¹⁸⁶Re, ¹⁸⁸Re²⁰³Pb, ⁶⁷Ga, ⁶⁸Ga, ⁴³Sc, ⁴⁷Sc, ^{110m}In, ¹¹¹Iₙ, ⁹⁷Ru, ⁶²Cu, ⁶⁴Cu, ⁶⁷Cu, ⁶⁸Cu, ⁸⁶Y, ⁹⁰Y⁸⁸Y, ¹²¹Sn, ¹⁵³Sm, ¹⁶⁶Ho, ¹⁰⁵Rh, ¹⁷⁷Lu_{,} ¹⁷²Lu, ²¹¹At and / or ¹⁸F or a mixture thereof.

Especially preferred is the use of ¹³¹I, ¹²⁵I, and ¹⁷⁷Lu_{.}

Most preferred is the use of ¹³¹I_{.}

In an especially preferred embodiment of the present invention, an ¹³¹I-labeled fusion protein is used, wherein the fusion protein in its monomeric form contains a V1 domain of L19, a Vh domain of L19 and an εₛ₂-CH4 domain.

Most preferred is the use of ¹³¹I labelled L19-SIP.

Most preferred is the use of Cetuximab.

In an especially preferred embodiment, the combination comprises ¹³¹I, labelled L19-SIP and an anti-EGFR-antibody, in particular Cetuximab (Erbitux).

Preferred is a combination according to the invention for use as medicament.

More preferred is a combination according to the invention for use as a medicament for treatment of cancer.

In another embodiment, the invention relates to a combination according to the invention, wherein the cancer is selected from head and neck cancer, non-small cell lung cancer, prostate cancer, colorectal cancer, ovarian cancer, pancreatic cancer, gastric cancer, and / or breast cancer.

"Specifically recognising" according to the present invention refers to antibody binding to a predetermined antigen. Typically, the antibody binds with an affinity of at least about 1x 10⁷ M⁻¹, and binds to the predetermined antigen with an affinity that is at least two-fold greater than its affinity for binding to a non-specific antigen (e.g., BSA, casein) other than the predetermined antigen or a closely-related antigen. The antigen is the ED-B domain of fibronectin.

Antibody linker is any linker, preferably a peptide linker, which is suitable for linking Vh and V1 domains. Suitable linkers are for example described in Bird et al, Science, 242, 423-426, 1988; Huston et al, PNAS USA, 85, 5879-5883, 1988, EP 0 573 551; EP 0 623679 and EP 0 318554.

Fusion protein linkers are linkers suitable for linking an antibody part according to the invention to the εs2-CH4 domain. A suitable linker is shown in SEQ ID No. 5.

εₛ₂-CH4 part is defined in Erqiu Li et al. "Mammalian cell expression of dimeric small immunue proteins (SIP)" (1997) Protein Engineering Vol.: 10, no. 6 pages 731 - 736. The sequence of is shown in SEQ ID No. 4.

**"Antibody-dependent cell-mediated cytotoxicity"** and "ADCC" refers to a cell-mediated reaction in which non-specific cytotoxic cells that express Fc receptors (FcRs) (*e.g*. Natural Killer (NK) cells, neutrophils, and macrophages) recognise bound antibody on a target cell and subsequently cause lysis of the target cell. The primary cells for mediating ADCC, NK cells, express FcyRIII only, whereas monocytes express FcγRI, FcyRII and FcγRIII. FcR expression on haematopoietic cells in summarized is Table 3 on Page 464 of Ravetch and Kinet, Annu. Rev. Immuno19:457-92 (1991*).* To assess ADCC activity of a molecule of interest, an *in vitro* ADCC assay, such as that described in US Patent No. 5,500,362 or 5,821,337 may be performed. Useful effector cells for such assays include peripheral blood mononuclear cells (PBMC) and Natural Killer (NK) cells. Alternatively, or additionally, ADCC activity of the molecule of interest may be assessed *in vivo, e.g.,* in a animal model such as that disclosed in Clynes et al. PNAS (USA) 95: 652-656 (1998*).*

**"Human effector cells"** are leukocytes which express one or more FcRs and perform effector functions. Preferably, the cells express at least FcγRIII and carry out ADCC effector function. Examples of human leukocytes which mediate ADCC include peripheral blood mononuclear cells (PBMC), natural killer (NK) cells, monocytes, cytotoxic T cells and neutrophils; with PBMCs and NK cells being preferred.

The terms **"Fc receptor" or "FcR"** are used to describe a receptor that binds to the Fc region of an antibody. The preferred FcR is a native sequence human FcR. Moreover, a preferred FcR is one which binds an IgG antibody (a gamma receptor) and includes receptors of the FcγRI, FcγRII, and FcγRIII subclasses, including allelic variants and alternatively spliced forms of these receptors. FcγRII receptors include FcγRIIA (an "activating receptor") and FcγRIIB (an "inhibiting receptor"), which have similar amino acid sequences that differ primarily in the cytoplasmic domains thereof. Activating receptor FcγRIIA contains an immunoreceptor tyrosine-based activation motif (ITAM) in its cytoplasmic domain. Inhibiting receptor FcγRIIB contains an immunoreceptor tyrosine-based inhibition motif (ITIM) in its cytoplasmic domain. (See Daeron, Annu. Rev. Immunol. 15:203-234 (1997*)).* FcRs are reviewed in Ravetch and Kinet, Annu. Rev. Immunol9:457-92 (1991*):* Capel et al., Immunomethods 4:25-34 (1994*);* and de Haas et al., J. Lab. Clin. Med. 126:330-41 (1995*).* Other FcRs, including those to be identified in the future, are encompassed by the term "FcR" herein. The term also includes the neonatal receptor, FcRn, which is responsible for the transfer of maternal IgGs to the foetus (Guyer et al., J. Immunol. 11 7587 (1976*)* and Kim et al., J. Immunol. 24: 249 (1994*)).*

**The Antibody - part** according to the present invention is understood as single chain Fv antibody fragment (scFv) of an antibody. Preferably, the antibody part is human, chimeric or humanized, particularly preferred human.

The term **"antibody"** herein is used in the broadest sense and specifically covers intact monoclonal antibodies, polyclonal antibodies, multispecific antibodies (*e.g*. bispecific antibodies) formed from at least two intact antibodies, and antibody fragments so long as they exhibit the desired biological activity.

**"Antibody fragments"** comprise a portion of an intact antibody, preferably comprising the antigen-binding or variable region thereof. Examples of antibody fragments include Fab, Fab', F(ab')₂ and Fv fragments; diabodies; linear antibodies; single-chain antibody molecules; and multispecific antibodies formed from antibody fragments.

**"Native antibodies"** are usually heterotetrameric glycoproteins of about 150,000 daltons, composed of two identical light (L) chains and two identical heavy (H) chains.

Each light chain is linked to a heavy chain by one covalent disulfide bond, while the number of disulfide linkages varies among the heavy chains of different immunoglobulin isotypes. Each heavy and light chain also has regularly spaced intrachain disulfide bridges. Each heavy chain has at one end a variable domain (V_{H}) followed by a number of constant domains. Each light chain has a variable domain at one end (V_{L}) and a constant domain at its other end; the constant domain of the light chain is aligned with the first constant domain of the heavy chain, and the light-chain variable domain is aligned with the variable domain of the heavy chain. Particular amino acid residues are believed to form an interface between the light chain and heavy chain variable domains.

The term **"variable"** refers to the fact that certain Portions of the variable domains differ extensively in sequence among antibodies and are used in the binding and specificity of each particular antibody for its particular antigen. However, the variability is not evenly distributed throughout the variable domains of antibodies. It is concentrated in three segments called hypervariable regions both in the light chain and the heavy chain variable domains. The more highly conserved portions of variable domains are called the framework regions (FRs). The variable domains of native heavy and light chains each comprise four FRs, largely adopting a β-sheet configuration, connected by three hypervariable regions, which form loops connecting, and in some cases forming part of, the β-sheet structure. The hypervariable regions in each chain are held together in close proximity by the FRs and, with the hypervariable regions from the other chain, contribute to the formation of the antigen-binding site of antibodies (See Kabat et al., Sequences of Proteins Immunological Interest, 5th Ed. Public Health Service, National Institutes of Health, Bethesda, MD. (1991)). The constant domains are not involved directly in binding an antibody to an antigen, but exhibit various effector functions, such as participation of the antibody in antibody dependent cellular cytotoxicity (ADCC).

Papain digestion of antibodies produces two identical antigen-binding fragments, called **"Fab" fragments,** each with a Single antigen-binding site, and a residual "Fc" fragment, whose name reflects its ability to crystallize readily. Pepsin treatment yields an F(ab')₂ fragment that has two antigen-binding sites and is still capable of cross-linking antigen.

**"Fv"** is the minimum antibody fragment which contains a complete antigen-recognition and antigen-binding site. This region consists of a dimer of one heavy chain and one light chain variable domain in tight, non-covalent association. It is in this configuration that the three hypervariable regions of each variable domain interact to define an antigen-binding site on the surface of the V_{H}-V_{L} dimer. Collectively, the six hypervariable regions confer antigen-binding specificity to the antibody. However, even a single variable domain (or half of an Fv comprising only three hypervariable regions specific for an antigen) has the ability to recognize and bind antigen, although at a lower affinity than the entire binding site.

The Fab fragment also contains the constant domain of the light chain and the first constant domain (CH1) of the heavy chain. Fab' fragments differ from Fab fragments by the addition of a few residues at the carboxy terminus of the heavy chain CH1 domain including one or more cysteines from the antibody hinge region. Fab'-SH is the designation herein for Fab' in which the cysteine residue(s) of the constant domains bear at least one free thiol group. F(ab'), antibody fragments originally were produced as pairs of Fab'₂-fragments which have hinge cysteines between them. Other chemical couplings of antibody fragments are also known.

The **"light chains"** of antibodies (immunoglobulins) from any vertebrate species can be assigned to one of two clearly distinct types, called kappa (κ) and lambda (λ), based on the amino acid sequences of their constant domains.

Depending on the amino acid sequence of the constant domain of their heavy chains, antibodies can be assigned to different classes. There are five major classes of intact antibodies: IgA, IgD, IgE, IgG, and IgM, and several of these may be further divided into subclasses (isotypes), *e.g*., IgG1, IgG2, IgG3, IgG4, IgA, and IgA2. The heavy-chain constant domains that correspond to the different classes of antibodies are called α, δ ε, γ, and µ respectively. The subunit structures and three-dimensional configurations of different classes of immunoglobulins are well known.

" **Single-chainFv" or' scFv** " **antibody fragments** comprise the V_{H} and V_{L} domains of antibody, wherein these domains are present in a Single Polypeptide chain. Preferably, the Fv Polypeptide further comprises a Polypeptide linker between the V_{H} and V_{L}, domains which enables the scFv to form the desired structure for antigen binding. For a review of scFv See Plückthun in The Pharmacology of Monoclonal Antibodies, vol. 113, Rosenburg and Moore eds., Springer-Verlag, New York, pp. 269-3 15 (1994).

In a further embodiment, antibodies or **antibody fragments can be isolated from antibody phage libraries generated** using the techniques described in McCafferty et al., Nature, 348552-554 (1990). Clackson et al., Nature, 352:624-628 (1991) and Marks et al., J Mol. Biol., 222581-597 (1991) describe the isolation of murine and human antibodies, respectively, using phage libraries. Subsequent publications describe the production of high affinity (mM range) human antibodies by chain shuffling (Marks et al., Bio/Technology, 10:779-783 (1992)), as well as combinatorial infection and *in vivo* recombination as a strategy for constructing very large phage libraries (Waterhouse et al., Nuc. Acids. Res., 21:2265-2266 (1993)). Thus, these techniques are viable alternatives to traditional monoclonal antibody hybridoma techniques for isolation of monoclonal antibodies. The DNA also may be modified, for example, by substituting the coding sequence for human heavy- and light-chain constant domains in place of the homologous murine sequences (U.S. Patent No. 4,8 16,567; Morrison, et al., Proc. Nat. Acad Sci. USA, 8 1 :685 1 (1984)), or by covalently joining to the immunoglobulin coding sequence all or part of the coding sequence for a non-immunoglobulin polypeptide. Typically such non-immunoglobulin polypeptides are substituted for the constant domains of an antibody, or they are substituted for the variable domains of one antigen-combining site of an antibody to create a chimeric bivalent antibody comprising one antigen-combining site having specificity for an antigen and another antigen-combining site having specificity for a different antigen.

The term **"diabodies"** refers to small antibody fragments with two antigen-binding sites, which fragments comprise a heavy-chain variable domain (V_{H}) connected to a light-chain variable domain (V_{L}) in the same polypeptide chain (V_{H} - V_{L}). By using a linker that is too short to allow pairing between the two domains on the same chain, the domains are forced to pair with the complementary domains of another chain and create two antigen-binding sites. Diabodies are described more fully in, for example, EP 0 404 097; WO 93/11161; and Hollinger et al., Proc. Natl. Acad. Sci. USA, 905444-6448 (1993).

The term **"monoclonal antibody"** as used herein refers to an antibody obtained from a population of substantially homogeneous antibodies, *i.e*., the individual antibodies comprising the population are identical except for possible naturally occurring mutations that may be present in minor amounts. Monoclonal antibodies are highly specific, being directed against a Single antigenic site. Furthermore, in contrast to conventional (polyclonal) antibody preparations which typically include different antibodies directed against different determinants (epitopes), each monoclonal antibody is directed against a single determinant on the antigen. In addition to their specificity, the monoclonal antibodies are advantageous in that they are synthesized by the hybridoma culture, uncontaminated by other immunoglobulins. The modifier "monoclonal" indicates the character of the antibody as being obtained from a substantially homogeneous population of antibodies, and is not to be construed as sequiring production of the antibody by any particular method. For example, the monoclonal antibodies to be used in accordance with the present invention may be made by the hybridoma method first described by Kohler et al., Nature, 256: 495 (1975), or may be made by recombinant DNA methods (See, *e.g*., U.S. Patent No. 4,816,567).

The **"monoclonal antibodies" may also be isolated from phage antibody libraries** using the techniques described in Clackson et al., Nature, 352:624-628 (1991) and Marks et al., J. Mol. Biol., 222581-597 (1991), for example.

The monoclonal antibodies herein specifically include **"chimeric" antibodies** (immunoglobulins) in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit the desired biological activity (U.S. Patent No. 4,s 16,567; Morrison et al., Proc. Natl. Acad Sci. USA, 8 1 :685 1-6855 (1984)).

Chimeric antibodies of interest herein include **"primatised" antibodies** comprising variable domain antigen-binding sequences derived from a non-human primate (*e.g*. Old World Monkey, such as baboon, rhesus or cynomolgus monkey) and human constant region sequences (US Pat No. 5,693,780).

**Humanised antibodies:** Methods for humanising non-human antibodies have been described in the art. Preferably, a humanised antibody has one or more amino acid residues introduced into it from a source which is non-human. These non-human amino acid residues are often referred to as."import" residues, which are typicality taken from an "import" variable domain. Humanisation can be essentially performed following the method of Winter and Co-workers *(*Jones et al., Nature, 321522-525 (1986); Riechmann et al., Nature, 332:323-327 (1988); Verhoeyen et al., Science, 239: 1534- 1536 (1988)), by substituting hypervariable region sequences for the corresponding sequences of a human antibody. Accordingly, such "humanised" antibodies are chimeric antibodies (U.S. Patent No. 4,8 16,567) wherein substantially less than an intact human variable domain has been substituted by the corresponding sequence from a non-human species. In practice, humanised antibodies are typically human antibodies in which some hypervariable region residues and possibly some FR residues are substituted by residues from analogous sites in rodent antibodies. The choice of human variable domains, both light and heavy, to be used in making the humanized antibodies is very important to reduce antigenicity. According to the so-called "best-fit" method, the sequence of the variable domain of a rodent antibody is screened against the entire library of known human variable-domain sequences. The human sequence which is closest to that of the rodent is then accepted as the human framework region (FR) for the humanised antibody (Sims et al., J. Immunol., 151:2296 (1993); Chothia et al., J. Mol. Biol., 196:901 (1987)). Another method uses a particular framework region derived from the consensus sequence of all human antibodies of a particular subgroup of light or heavy chains. The Same framework may be used for several different humanised antibodies (Carter et al., Proc. Natl. Acad Sci. USA, 89:4285 (1992); Presta et al., J. Immunol., 151:2623 (1993)). It is further important that antibodies be humanized with retention of high affinity for the antigen and other favourable biological properties. To achieve this goal, according to a preferred method, humanised antibodies are prepared by a process of analysis of the parental sequences and various conceptual humanised products using three - dimensional models of the parental and humanized sequences. Three - dimensional immunoglobulin models are commonly available and are familiar to those skilled in the art. Computer programs are available which illustrate and display probable three-dimensional conformational structures of selected candidate immunoglobulin sequences.

Inspection of these displays permits analysis of the likely role of the residues in the functioning of the candidate immunoglobulin sequence, *i. e*., the analysis of residues that influence the ability of the candidate immunoglobulin to bind its antigen. In this way, FR residues can be selected and combined from the recipient and import sequences so that the desired antibody characteristic, such as increased affinity for the target antigen(s), is achieved. In general, the hypervariable region residues are directly and most substantially involved in influencing antigen binding.

**Human antibodies:** As an alternative to humanisation, human antibodies can be generated. For example, it is now possible to produce transgenic animals (*e.g*., mice) that are capable, upon immunisation, of producing a full repertoire of human antibodies in the absence of endogenous immunoglobulin production. For example, it has been described that the homozygous deletion of the antibody heavy-chain joining region (JH) gene in chimeric and germ-line mutant mice results in complete inhibition of endogenous antibody production. Transfer of the human germ-line immunoglobulin gene array in such germ-line mutant mice will result in the production of human antibodies upon antigen challenge. See, e.g., Jakobovits et al., Proc. Natl. Acad. Sci. USA, 90:2551 (1993); Jakobovits et al., Nature, 362:255-258 (1993); Bruggermann et al., Year in Immuno., 7:33 (1993); and US Patent Nos. 5,591,669,5,589,369 and5,545,807.

Alternatively, **phage display technology** (McCafferty et al., Nature 348:552-553 (1990)) can be used to produce human antibodies and antibody fragments in vitro, from immunoglobulin variable (V) domain gene repertoires from unimmunized donors. According to this technique, antibody V domain genes are cloned in-frame into either a major or minor coat protein gene of a filamentous bacteriophage, such as M 13 or fd, and displayed as functional antibody fragments on the surface of the phage particle. Because the filamentous particle contains a single-stranded DNA copy of the phage genome, selections based on the functional properties of the antibody also result in selection of the gene encoding the antibody exhibiting those properties. Thus, the phage mimics some of the properties of the B cell. Phage display can be performed in a variety of formats; for their review See, e.g., Johnson, Kevin S. and Chiswell, David J., Current Opinion in Structural Biology 3:564-571(1993). Several sources of V-gene Segments can be used for phage display. Clackson et al., Nature, 352:624-628 (1991) isolated a diverse array of anti-oxazolone antibodies from a small random combinatorial library of V genes derived from the Spleens of immunized mice. A repertoire of V genes from unimmunized human donors can be constructed and antibodies to a diverse array of antigens (including self-antigens) can be isolated essentially following the techniques described by Marks et al., J. Mol. Biol. 222581-597 (1991), or Griffith et al., EMBO J. 12:725-734 (1993). See, also, US Patent Nos. 5,565,332 and 5,573,905. Human antibodies may also be generated by in vitro activated B cells (see US Patents 5,567,610 and 5,229,275).

The term **"hypervariable region"** when used herein refers to the amino acid residues of an antibody which are responsible for antigen-binding. The hypervariable region comprises amino acid residues from a "complementarily determining region" or "CDR" (*e.g.* residues 24-34 (L1), 50-56 (L2) and 89-97 (L3) in the light chain variable domain and 31-35 (H1), 50-65 (H2) and 95-102 (H3) in the heavy chain variable domain; Kabat et al., Sequences of Proteins of Immunological Interest, 5th Ed. Public Health Service, National Institutes of Health, Bethesda, MD. (1991)) and/or those residues from a "hypervariable loop" (*e.g*. residues 26-32 (L1), 50-52 (L2) and 91-96 (L3) in the light chain variable domain and 26-32 (H1), 53-55 (H2) and 96-101 (H3) in the heavy chain variable domain; Chothia and Lesk J Mol. Biol. 196:901-917 (1987)). "Framework" or "FR" residues are those variable domain residues other than the hypervariable region residues as herein defined.

**Antibody fragments:** Various techniques have been developed for the production of antibody fragments. Traditionally, these fragments were derived via proteolytic digestion of intact antibodies (See, e.g., Morimoto et al., Journal of Biochemical und Biophysical Methods 24: 107-1 17 (1992) and Brennan et al., Science, 229:81 (1985)). However, these fragments can now be produced directly by recombinant host cells. For example, the antibody fragments can be isolated from the antibody phage libraries discussed above. Alternatively, Fab'-SH fragments can be directly recovered from E. coli and chemically coupled to form F(ab')₂ fragments (Carter et al., Biotechnology 10: 163- 167 (1992)). According to another approach, F(ab')₂ fragments can be isolated directly from recombinant host cell culture. Other techniques for the production of antibody fragments will be apparent to the skilled practitioner. In other embodiments, the antibody of choice is a single chain Fv fragment (scFv). See WO 93/16185; US Patent NO. 5,571,894; and US Patent No. 5,587,458. The antibody fragment may also be a "linear antibody", e.g., as described in US Patent 5,641,870 for example. Such linear antibody fragments may be monospecific or bispecific.

The term "specifically recognising" refers to antibody binding to a predetermined antigen. Typically, the antibody binds with an affinity of at least 1x10⁻⁷ M, and binds to the ED-B domain of fibronectin that is at least two-fold greater than the affinity for binding to a non-specific antigen (e.g. BSA, casein) other than ED-B domain of fibronectin.

**Amino acid sequence modification(s) of protein or Peptide antagonists or antibody** - **part** described herein are contemplated. For example, it may be desirable to improve the binding affinity and/or other biological properties of the antagonist. Amino acid sequence variants of the antagonist are prepared by introducing appropriate nucleotide changes into the antagonist nucleic acid, or by peptide synthesis. Such modifications include, for example, deletions from, and/or insertions into and or Substitutions of, residues within the amino acid sequences of the antagonist. Any combination of deletion, insertion, and substitution is made to arrive at the final construct, provided that the final construct possesses the desired characteristics. Therefore, in the case of the heavy of light chain a variation of 1; 2; 3; 4; 5; 6; 7; 8; 9; 10; 11; 12; 13; 14; 15; 16; 17; 18; 19: or 20 amino acids can be executed. In the case of εₛ₂ -CH4 a variation of 1; 2; 3; 4; 5; 6; 7; 8; 9; 10; 11; 12; 13; 14; 15; 16; 17; 20; 21; 22; 23; 24; 25; 26; 27; 28; 29; 30; 31; 32; or 33 amino acids can be executed. In the case of a linker a variation of 1; 2; 3; 4; 5; 6; or 7 amino acids can be executed. In case of the linker the variations are much more flexible, because function is simple to create a sufficient space between the functional amino acid sequences. A variation is defined as a deletion, insertion and/or substitution.

The amino acid changes also may alter post-translational processes of the antagonist, such as changing the number or position of glycosylation sites. A useful method for identification of certain residues or regions of the antagonist that are preferred locations for mutagenesis is called "alanine scanning mutagenesis" as described by Cunningham and Welk Science, 244: 108 1-1085 (1989). Here, a residue or group of target residues are identified (*e.g*., charged residues such as Arg, Asp, His, Lys, and Glu) and replaced by a neutral or negatively charged amino acid (most preferably alanine or polyalanine) to affect the interaction of the amino acids with antigen. Those amino acid locations demonstrating functional sensitivity to the substitutions then are refined by introducing further or other variants at, or for, the sites of substitution. Thus, while the site for introducing an amino acid sequence variation is predetermined, the nature of the mutation *per se* need not be predetermined. For example, to analyze the performance of a mutation at a given site, Ala scanning or random mutagenesis is conducted at the target codon or region and the expressed antagonist variants are screened for the desired activity. Amino acid sequence insertions include amino- and/or carboxyl-terminal fusions ranging in length from one residue to Polypeptides containing a hundred or more residues, as well as intrasequence insertions of single or multiple amino acid residues. Examples of terminal insertions include an antagonist with an N-terminal methionyl residue or the antagonist fused to a cytotoxic polypeptide. Other insertional variants of the antagonist molecule include the fusion to the N- or C-terminus of the antagonist of an enzyme, or a polypeptide which increases the serum half-life of the antagonist. Another type of variant is an amino acid substitution variant. These variants have at least one amino acid residue in the antagonist molecule replaced by a different residue. The sites of greatest interest for substitutional mutagenesis of antibody antagonists include the hypervariable regions, but FR alterations are also contemplated. Conservative substitutions are shown in Table 1 under the heading of "preferred substitutions".

**Table 1**

| **Original Residue** | **Exemplary substitution** | **Preferred Substitution** |
|---|---|---|
| Ala | Val; Leu; Ile | Val |
| Arg | Lys, Gln, Asn | Lys |
| Asn | Gln, His, Asp, Lys, Arg | Gln |
| Asp | Glu, Asn | Glu |
| Cys | Ser, Ala | Ser |
| Gln | Asn, Glu | Asn |
| Glu | Asp, Gln, | Asp |
| Gly | Ala | Ala |
| His | Asn, Gln, Lys, Arg | Arg |
| Ile | Leu, Val, Met, Ala, Phe, Norleucine | Leu |
| Leu | Norleucine, Ile, Val, Met, Ala, Phe | Ile |
| Lys | Arg, Gln, Asn | Arg |
| Met | Leu, Phe, Ile, | Leu |
| Phe | Leu, Val, Ile, Ala, Tyr | Tyr |
| Pro | Ala | Ala |
| Ser | Thr | Thr |
| Thr | Ser | Ser |
| Trp | Tyr, Phe | Tyr |
| Tyr | Trp, Phe, Thr, Ser | Phe |
| Val | Ile, Leu, Met, Phe, Ala, Norleucine | Leu |

If such substitutions result in a change in biological activity, then more substantial changes, denominated "exemplary substitutions" in Table 1, or as further described below in reference to amino acid classes, may be introduced and the products screened.

Substantial modifications in the biological properties of the antagonist are accomplished by selecting substitutions that differ significantly in their effect on maintaining (a) the structure of the polypeptide backbone in the area of the substitution, for example, as a sheet or helical conformation, (b) the charge or hydrophobicity of the molecule at the target site, or (c) the bulk of the side chain. Naturally occurring residues are divided into groups based on common side-chain properties:
(1) hydrophobic: Norleucine, Met, Ala, Val, Leu, Ile;
(2) neutral hydrophilic: Cys, Ser, Thr;
(3) acidic: Asp, Glu;
(4) basic: Asn, Gln, His, Lys, Arg;
(5) residues that influence chain orientation: Gly, Pro; and
(6) aromatic: Trp, Tyr, Phe.

Non-conservative substitutions will entail exchanging a member of one of these classes for another class. Any cysteine residue not involved in maintaining the proper conformation of the antagonist also may be substituted, generally with serine, to improve the oxidative stability of the molecule and prevent aberrant cross linking. Conversely, cysteine bond(s) may be added to the antagonist to improve its stability (particularly where the antagonist is an antibody fragment such as an Fv fragment).

A particularly preferred type of substitutional variant involves substituting one or more hypervariable region residues of a parent antibody. Generally, the resulting variant(s) selected for further development will have improved biological properties relative to the parent antibody from which they are generated. A convenient way for generating such substitutional variants is affinity maturation using phage display. Briefly, several hypervariable region sites (*e.g*. 6-7 sites) are mutated to generate all possible amino substitutions at each site. The antibody variants thus generated are displayed in a monovalent fashion from filamentous phage particles as fusions to the gene III product of M13 packaged within each particle. The phage-displayed variants are then screened for their biological activity (*e.g*. binding affinity) as herein disclosed. In order to identify candidate hypervariable region sites for modification, alanine scanning mutagenesis can be performed to identify hypervariable region residues contributing significantly to antigen binding. Alternatively, or in additionally, it may be beneficial to analyse a crystal structure of the antigen-antibody complex to identify contact points between the antibody and antigen. Such contact residues and neighbouring residues are candidates for substitution according to the techniques elaborated herein. Once such variants are generated, the panel of variants is subjected to screening as described herein and antibodies with superior properties in one or more relevant assays may be selected for further development.

Another type of amino acid variant of the antagonist alters the original glycosylation pattern of the antagonist. By altering is meant deleting one or more carbohydrate moieties found in the antagonist, and/or adding one or more glycosylation sites that are not present in the antagonist.

Glycosylation of polypeptides is typically either N-linked or O-linked. N-linked refers to the attachment of the carbohydrate moiety to the side chain of an asparagine residue. The tri - peptide sequences asparagine-X-serine and asparagine-X-threonine, where X is any amino acid except proline, are the recognition sequences for enzymatic attachment of the carbohydrate moiety to the asparagine side chain. Thus, the presence of either of these tri - peptide sequences in a polypeptide creates a potential glycosylation site. O-linked glycosylation refers to the attachment of one of the sugars N-aceylgalactosamine, galactose, or xylose to a hydroxyamino acid, most commonly serine or threonine, although 5-hydroxyproline or 5-hydroxylysine may also be used.

Addition of glycosylation sites to the antagonist is conveniently accomplished by altering the amino acid sequence such that it contains one or more of the above-described tri - peptide sequences (for N-linked glycosylation sites). The alteration may also be made by the addition of, or substitution by, one or more serine or threonine residues to the sequence of the original antagonist (for O-linked glycosylation sites).

Nucleic acid molecules encoding amino acid sequence variants of the antagonist are prepared by a variety of methods known in the art. These methods include, but are not limited to, isolation from a natural source (in the case of naturally occurring amino acid sequence variants) or preparation by oligonucleotide-mediated (or site-directed) mutagenesis, PCR mutagenesis, and cassette mutagenesis of an earlier prepared variant or a non-variant version of the antagonist.

It may be desirable to modify the antagonist of the invention with respect to effector function, *e.g*. so as to enhance antigen-dependent cell-mediated cyotoxicity (ADCC) and/or complement dependent cytotoxicity (CDC) of the antagonist. This may be achieved by introducing one or more amino acid substitutions in an Fc region of an antibody antagonist. Alternatively or additionally, cysteine residue(s) may be introduced in the Fc region, thereby allowing interchain disulfide bond formation in this region. The homodimeric antibody thus generated may have improved internalization capability and/or increased complement-mediated cell killing and antibody-dependent cellular cytotoxicity (ADCC). See Caron et al., J. Exp Med. 176:1191-1195 (1992) and Shopes, B. J. Immunol. 148:2918-2922 (1992). Homodimeric antibodies with enhanced anti-tumour activity may also be prepared using heterobifunctional cross-linkers as described in Wolff et al. Cancer Research 53:2560-2565 (1993). Alternatively, an antibody can be engineered which has dual Fc regions and may thereby have enhanced complement lysis and ADCC capabilities. See Stevenson et al. Anti-Cancer Drug Design 3:219-230 (1989).

Variations of the fusion protein L19-SIP are defined by variations as mentioned before. That means the variations of special sequences, mentioned in the following list of Table 2, which can be modified be deletion, insertion and / or substitution by the following numbers of amino acids:

**Table 2**

| Seq. Id No | Type | Numbers of amino acids, which may be deleted, inserted and / or substituted compared to SEQ ID. No. 1, 2, 3, 4, and 5. |
|---|---|---|
| Seq. Id No 1 | VH | 1, 2 ,3, 4, 5, 6, 7, 8, 9, 10, 11, 12, 13, 14, 15, 16, 17, 18 |
| Seq. Id No 2 | VL | 1, 2 , 3, 4, 5, 6, 7, 8, 9, 10, 11, 12, 13, 14, 15, 16, 17, 18 |
| Seq. Id No 3 | mAb linker | 1, 2 , 3; 4,5, 6, 7 |
| Seq. Id No 4 | εₛ₂-CH4 | 1, 2 ,3, 4, 5, 6, 7, 8, 9, 10, 11, 12, 13, 14, 15, 16 17, 18,19,20,21, 22,23,24,25, 26,27,28,29,30 |
| Seq. Id No 5 | Fusion protein linker | 1, 2 ,3 |

Further modifications are mentioned in Pini et al. (1998) J Biol. Chem., Vol.: 273, pages 21769 - 21776, WO 02 / 20563, WO 2005/37312, and WO 99 / 58570

Many appropriate imaging and radiotherapeutic agents are known in the art, as are methods for their attachment to antibody parts, fusion proteins, antibodies and binding ligand (see e.g., US 5,021,236 and US 4,472,509). Certain attachment methods involve the use of a metal chelate complex employing, for example, an organic chelating agent such a DTPA attached to the antibody (US 4,472,509). Radioactively labelled fusion proteins and antibody parts can also be iodinated by contact with sodium or potassium iodide and a chemical oxidising agent such as sodium hypochlorite [Redshaw M.R., Lynch S.S., J. Endochrinol., 60, 527 (1974)], or an enzymatic oxidizing agent, such as lactoperoxidase [Marchalonis J.J., Biochem. J., 113, 299 (1969)]. The proteins of the inventions may be labelled with technetium - 99 by a ligand exchange process, for example, by reducing pertechnetate with stannous solution, chelating the reduced technetium onto a sephadex column and applying the antibody to this column. Direct labelling techniques are also suitable, e.g., by incubation pertechnetate, a reducing agent such as SnCl₂, a buffer solution such as sodium - potassium phthalate solution, and the protein.

### Use as a medicament for radiotherapy/chemotherapy

The combination of the invention exhibits pharmacological activity and is, therefore, useful as pharmaceuticals. In particular, the combination shows pharmacological activity in a number of pathological or disease states in connection with a cancer, in particular head and neck cancer, non-small cell lung cancer, prostate cancer, colorectal cancer, ovarian cancer, pancreatic cancer, gastric cancer, and / or breast cancer.

The combination of the invention is therefore indicated for use in treatment of head and neck cancer, non-small cell lung cancer, prostate cancer, colorectal cancer, ovarian cancer, pancreatic cancer, gastric cancer, and / or breast cancer, especially pancreatic cancer. The fusion protein and an anti-EGFR-antibody may be administered simultaneously or separately, at different times.

For the treatment of such conditions, the appropriated dosage will, of course, vary depending upon, for example, the host, the mode of administration and the nature and severity of the condition being treated.

The dosage of an anti-EGFR-antibody administered will vary according to the mode of use and route of use, as well as to the requirements of the patient. In general, such antibody may be applied on a daily or weekly basis or in intervals of two of more days or weeks . For once-weekly application the standard dose for Erbitux is 250 mg/m² as an intravenous infusion over 60 minutes, and the starting dose is 400 mg/m² over 120 minutes. Thus, a starting dose, if necessary, for an anti-EGFR-antibody may be about 50 to 5000 mg/m² for about 10 minutes to several hours and a maintenance dose may be about 10 to 5000 mg/m² for an about 10 minutes to several hours infusion, repeated two or more times in intervals of 1 day to several days or 1 weeks or several weeks.

The dosage of the fusion protein according to claim 1, in particular radiolabelled L19-SIP, administered will vary according to the mode of use and route of use, as well as to the requirements of the patient. In general, a single dosage in man is in the range of about 2 - 200 MBq/kg body weight (10 -1,000 µg/kg body weight). The radio - labelled fusion protein must be given in a dose that does not cause toxicity of the most radiosensitive organ in the body (dose limiting organ). In animal experiments the red bone marrow was identified as dose limiting organ for ¹³¹I labelled antibodies. For proteins labelled with a radio - metal the kidney can be the dose limiting organ.

The preferred method of administration is parenteral, in particular intravenous infusion.

The invention also discloses a method of treating cancer in a patient in need of such treatment which method includes the administration of a combination of the present invention.

The invention also discloses a method of treating cancer in a patient in need of such treatment which method includes the administration of at least one fusion protein of the invention and an anti-EGFR-antibody.

The fusion protein and an anti-EGFR-antibody may be administered together, or separately, at the same time points or at different time points, as described above. Also, a signle administration of the combination is possible or administration of several doses.

SEQ ID No. 1 represents the V1 chain of the L19-SIP antibody part.

SEQ ID No. 2 represents the Vh chain of the L19-SIP antibody part.

SEQ ID No. 3 represents the antibody linker linking V1 and Vh the L19-SIP antibody part.

SEQ ID No. 4 represents the εₛ₂-CH4 domain part of L 19-SIP

SEQ ID No. 5 represents the linker linking the antibody part of L19-SIP and the εₛ₂-CH4 domain.

SEQ ID No. 6 to 11 represent the CDR sequences of the L19.

SEQ ID No. 12 represents the sequence of L19-SIP in its monomeric form.

SEQ ID No. 13 represents the sequence of an alternative antibody or fusion protein linker.
Figure 1: Bio - distribution of intravenously co - injected ¹²⁵I-L19-SIP (A) and 177Lu-L19-SIP (B) in FaDu xenograft bearing nude mice at 3, 6, 24, 48, 72, and 144 h after injection.
Figure 2: Bio - distribution of intravenously injected ¹³¹I-L19-SIP in FaDu xenograft bearing nude mice at 24, 48, and 72 h after injection.
Figure 3 Bio - distribution of intravenously injected ¹³¹I-L19-SIP in HNX-OE xenograft bearing nude mice at 24, 48, and 72 h after injection.
Figure 4: Mean body weights of FaDu xenograft bearing nude mice intra - peritoneally injected with diluent or increasing doses of ¹³¹I-L19-SIP. Values are the mean of 5 mice. Standard deviations have been omitted but were less than 5%.
Figure 5: Mean tumour volume (A) and survival (B) of FaDu xenograft bearing nude mice after intra - peritoneally injected of ¹³¹I-L19-SIP (RIT) at day 0 and/or Cetuximab (1 mg given two times a week i.p. for four weeks). Standard deviations have been omitted for sake of clarity.
Figure 6: Mean tumour volume (A) and survival (B) of HNX-OE xenograft bearing nude mice intra - peritoneally injected with ¹³¹I-L19-SIP (RIT) and/or Cetuximab (1 mg given two times a week i.p. for four weeks). Standard deviations have been omitted for sake of clarity.

### Examples

### MATERIALS AND METHODS

### Monoclonal antibody, Xenograft lines, and Radioactivity

Antibody L19-SIP (0.3 - 0.5 mg/ml), directed against the ED-B domain of fibronectin, was obtained from the Institute of Pharmaceutical Sciences, Swiss Federal Institute of Technology, Zurich, Switzerland. Selection, construction and production of L19-SIP (Mw = ~ 80 kD) have been described previously (Pini A, Viti F, Santucci A, et al. Design and use of a phage display library. Human antibodies with subnanomolar affinity against a marker of angiogenesis eluted from two-dimensional gel. J Biol Chem 1998;273:21769-76; Borsi L, Balza E, Bestagno M, et al. Selective targeting of tumoral vasculature: comparison of different formats of an antibody (L19) to the ED-B domain of fibronectin. Int J Cancer 2002;102:75-85). A schematic drawing of its molecular structure was provided by Berndorff et al. (Berndorff D, Borkowski S, Sieger S, et al. Radioimmunotherapy of solid tumors by targeting extra domain B fibronectin: Identification of the best-suited radioimmunoconjugate. Clin Cancer Res 2005;11:7053s-63s). Monoclonal antibody Cetuximab (C225/Erbitux^{®}, 2 mg/ml) was obtained from Merck. Cetuximab is a mouse-human chimeric anti-EGFR MAb that binds with high affinity to the receptor, blocks ligand-induced activation of receptor tyrosine kinase, and induces dimerization and down - regulation of EGFR (Mendelsohn J, Baselga J. Status of epidermal growth factor receptor antagonists in the biology and treatment of cancer. J Clin Oncol 2003;21:2787-99.). The human HNSCC cell lines FaDu and HNX-OE have been described before (Van Gog FB, Brakenhoff RH, Stigter-Van Walsum M, Snow GB, Van Dongen GAMS. Perspectives of combined radioimmunotherapy and anti-EGFR antibody therapy for the treatment of residual head and neck cancer. Int J Cancer 1998;77:13-8; Ranger SR. A new human cell line (FaDu) from a hypopharyngeal carcinoma. Cancer 1972;29:117-21.)
¹³¹I (7.4 GBq/ml) and 125I (3.7 GBq/ml) were purchased from Amersham Biosciences, while 177Lu (725 GBq/mg) was obtained from Perkin-Elmer.

Immuno - histochemical staining of HNSCC xenografts:

The two HNSCC xenograft lines FaDu and HNX-OE were characterized for ED-B and EGFR expression, by performing immuno - histochemistry with L19-SIP and Cetuximab, respectively. 5 µm-thick cryostat sections were air-dried and fixed in cold aceton for 10 min at -20°C,

For immuno - histochemical staining of ED-B, biotinylated L19-SIP was used in the first step, while the DAKO ChemMate™ Detection Kit (Streptavidin-Alkaline Phosphatase/Red, DakoCytomation) was used for colour development. In short, after fixation the sections were incubated in Tris buffer (50 mmol/1, pH 7.2) for 30 min, followed by incubation with biotinylated antibody L19-SIP for 1 h at room temperature (RT). After extensive washing with the Tris buffer (3 times for 5 min), sections were incubated with the ChemMate™ Detection Kit according to instructions provided by the supplier. After washing with water, hematoxylin (Merck) 1:4 in water was used as a counter - stain followed by mounting in Kaiser glycerine.

For immuno - histochemical staining of EGFR, Cetuximab was used as the primary antibody followed by horse radish peroxidase (HRP)-labelled rabbit anti-human IgG (Dako). After fixation sections were blocked with 2% bovine serum albumin (BSA) in PBS for 20 min at RT. Sections were incubated with Cetuximab 10 µg/ml for 1 h at RT. After extensive washing with PBS (3 times for 5 min), sections were incubated with rabbit anti-human IgG HRP 1:100 for 1 h at RT. After washing with PBS (3 times for 5 min), sections were incubated for 5 min with diaminobenzidine (DAB) 1:10 in PBS with 10 µl H₂O₂ for colour development. After washing with water, hematoxylin 1:4 in water was used as a counter - stain followed by mounting in Kaiser glycerine.

Staining intensity was evaluated semi-quantitatively.

### Radiolabeling for biodistribution experiments

Preparation of 125I-L19-SIP and ¹³¹I-L19-SIP. Iodination of L19-SIP with either 125I or ¹³¹I was performed essentially as described previously (Visser GWM, Klok RP, Klein-Gebbink JW, Ter Linde T, Van Dongen GAMS, Molthoff CF. Optimal quality Iodine-131-monoclonal antibodies upon high dose labeling in a large reaction volume and temporarily coating the antibody with iodogen. J Nucl Med 2001;42:509-519). In short, 20 ml β-scintillation glass vials were coated with 75 µg IODO-GEN (1,3,4,6-tetrachloro-3α,6α-diphenyl-glycouril; Pierce Biotechnology) in dichloromethane, dried under a stream of N2 gas, resulting in a thin coating of IODO-GEN on the bottom surface of the vial. The vials were stored under N2 atmosphere till use. To an IODO-GEN-coated glass vial, successively 50 µl Na2HPO4 (0.5 mol/l, pH 7.4), 72 - 150 µg L19-SIP in 450 µl Na2HPO4 (0.1 mol/l, pH 6.8) and 6.7 - 11.1 MBq 125I or ¹³¹I were added. After gentle shaking for 4 min at room temperature, 0.1 ml ascorbic acid (25 mg/ml, pH 5) was added. After an additional 5 min, the reaction mixture was transferred to a syringe connected to a filter (0.22 µm Acrodisc, Gelman Sciences) followed by 0.4 ml Na2HPO4 (0.1 mol/l, pH 6.8), used for an additional rinsing of the reaction vial. This combined solution was filtered and purified on a PD-10 column with 0.9% NaCl/ascorbic acid (5 mg/ml, pH 5.0, Bufa) as eluent. The first 2.5 ml (1.0 ml sample volume and the first 1.5 ml) were discarded and the radio - labelled L19-SIP was collected in the next 2.0 ml. Preparation of 177Lu-L19-SIP. Antibody L19-SIP was conjugated with p-isothiocyanatobenzyl-1,4,7,10-tetraazacyclododecane-1,4,7,10-tetraacetic acid (p-SCN-Bz-DOTA; Macrocyclics), essentially as described by Perk et al. (Perk LR, Visser GWM, Vosjan MJWD, et al. 89Zr as a PET surrogate radioisotope for scouting biodistribution of the therapeutic radiometals 90Y and 177Lu in tumor-bearing nude mice after coupling to the internalizing antibody cetuximab. J Nucl Med 2005;46:1898-906). All steps were performed under strict metal-free conditions. L19-SIP was extensively dialyzed (Slide-A-Lyzer dialysis cassettes, Pierce Biotechnology) against metal-free NaHCO3 (0.1 mol/l, pH 9.0) containing 2 g of Chelex 100 (Bio-Rad) per litre. For conjugation 125 µl p-SCN-Bz-DOTA (0.8 mg/ml in NaHCO3, 0.1 mol/l; pH 9.0) and 875 µl (0.438 mg) of L19-SIP were incubated for 30 min at 37oC, pH 9.0. The pre - modified L19-SIP was purified using a pre - washed PD-10 column and eluted with CH3COONH4 (0.25 mol/l, pH 5.5). Approximately one p-SCN-Bz-DOTA moiety was coupled per L19-SIP molecule. L19-SIP-p-SCN-Bz-DOTA (150 µg) was labelled with 177Lu (18.5 MBq) in a total volume of 700 µl CH3COONH4 (0.25 mol/l, pH 5.5). After labelling for 60 min at 45oC, 50 µl 0.05 M EDTA was added to the reaction vial, and the mixture was incubated for another 5 min. Unbound 177Lu was removed using a prewashed PD-10 column, and 177Lu-labeled L19-SIP was eluted with 0.9% NaCl. The first 3.0 ml (750 µL sample volume, 250 µl CH3COONH4 for rinsing and the first 2.0 ml) were discarded and the radio labelled L19-SIP was collected in the next 1.5 ml.

### Radio labelling for therapy experiments

Preparation of ¹³¹I-L19-SIP for therapy was performed according the so-called "IODO-GEN-coated MAb method", essentially as described previously (Visser GWM, Klok RP, Klein-Gebbink JW, Ter Linde T, Van Dongen GAMS, Molthoff CF. Optimal quality Iodine-131-monoclonal antibodies upon high dose labeling in a large reaction volume and temporarily coating the antibody with iodogen. J Nucl Med 2001;42:509-519). In short, ¹³¹I (884 - 1880 MBq) was added to a glass vial in 1 ml NaOH (1 mmol/l), after which 10 µl ascorbic acid (1,41 mg/ml, Bufa) was added. After gentle shaking for 1 min at RT, 400 µl phosphate buffer (0.5 mol/l, pH 7.2) was added, followed by 3 ml L19-SIP (0.9 - 1.5 mg) and 35 µL IODO-GEN/MeCN (1 mg/ml). After 3 min the reaction was stopped by adding 100 µl ascorbic acid (25 mg/ml). Five min later, 50 µl human serum albumin (20% HSA) was added. Another 4 min later the solution was purified on two PD-10 column with 0.9% NaCl/ascorbic acid (5 mg/ml, pH 5.0) as the eluent. For each column, the first 2.8 ml (2.3 ml sample volume and the first 0.5 ml) were discarded and the radio labelled L19-SIP was collected in the next 3.0 ml. For the MTD experiment 1 labelling sufficed, for therapy studies several labellings were performed, while product was pooled afterwards.

### Analysis

All conjugates were analyzed by instant thin layer chromatography (ITLC) for radiochemical purity, by high performance liquid chromatography (HPLC) and sodium dodecylsulfate-polyacrylamide gel electrophoresis (SDS-PAGE) followed by phosphor imager analysis for integrity, and by an antigen-binding assay for immunoreactivity. ITLC analysis of radio - labelled L19-SIP was carried out on silica gel impregnated glass fiber sheets (Gelman Sciences). As mobile phase, citrate buffer (20 mmol/l, pH 5.0) was used for ¹³¹I- and ¹²⁵I-labeled antibody. ITLC samples of ¹⁷⁷Lu-labeled L19-SIP were first incubated for 5 min in an EDTA solution (50 mmol/l) and subsequently spotted on ITLC. As the mobile phase, 0.9% NaCl was used. HPLC analysis of radio - labelled L19-SIP was performed as described previously (Perk LR, Visser GWM, Vosjan MJWD, et al. 89Zr as a PET surrogate radioisotope for scouting biodistribution of the therapeutic radiometals 90Y and 177Lu in tumor-bearing nude mice after coupling to the internalizing antibody cetuximab. J Nucl Med 2005;46:1898-906). The integrity of the radio - immuno - conjugates was monitored by electrophoresis on a Phastgel System (Amersham Biosciences) using preformed high density SDS-PAGE gels under non - reducing conditions and analyzed on a phosphor imager.

In vitro binding characteristics of radio - labelled L19-SIP were determined in an immuno - reactivity assay essentially as described previously (Birchler M, Neri G, Tardi L, Halin C, Viti F, Neri D. Infrared photodetection for the in vivo localization of phage-derived antibodies directed against angiogenic markers. J Immunol Methods 1999;231:239-48), using ED-B coated Sepharose resin in PBS/1% BSA. Five serial dilutions, ranging from 100 µL - 6.2 µl resin per tube, were prepared in triplicate in PBS/1% BSA. L19-SIP (125 ng) labelled with ¹²⁵I, ¹³¹I or ¹⁷⁷Lu was added to the tubes and the samples were incubated overnight at 4°C. Excess unlabeled L19-SIP antibody (10 µg per tube) was added to a second tube with the lowest concentration of resin to determine non-specific binding. Resin was spun down and radioactivity in the pellet and supernatant was determined in a γ-counter (Wallac LKB-CompuGamma 1282, Kabi Pharmacia) and the percentage bound and free radioactivity was calculated. Data were graphically analyzed in a modified Lineweaver Burk plot and the immunoreactive fraction was determined by linear extrapolation to conditions representing infinite antigen excess.

### Biodistribution of L19-SIP

For the biodistribution experiments, nude mice bearing subcutaneously implanted HNSCC xenograft lines FaDu or HNX-OE were used. Female mice (Hsd athymic nu/nu, 25-32 g; Harlan CPB) were 8-10 weeks old at the time of the experiments. All animal experiments were performed according to National Institutes of Health principles of laboratory animal care and Dutch national law ("Wet op de dierproeven", Stb 1985, 336).

Three biodistribution studies were performed. Biodistribution of coinjected ¹²⁵I-L19-SIP (0.37 MBq) and ¹⁷⁷Lu-L19-SIP (0.37 MBq) was assessed in FaDu bearing nude mice (n = 20) in the first experiment. In this experiment ¹²⁵I was used instead of ¹³¹I, to facilitate dual isotope counting with 177Lu. Biodistribution of ¹³¹I-L19-SIP (0.37 MBq) in FaDu and HNX-OE bearing nude mice (n = 12 each) was assessed in the second and third experiment, respectively. The tumour size at the start of the experiment was 83 ± 29, 73 ± 21, and 157 ± 95 mm3, in experiment 1, 2 and 3, respectively. Mice received 10 - 16 µg L19-SIP in a total volume of 150 µl i.v.. At 3, 6, 24, 48, 72 and 144 h (exp. 1) or 24, 48 and 72 h (exp. 2 and 3) after injection, groups of 3 - 4 mice containing 5 - 8 tumours were anaesthetized, bled, killed and dissected. Blood, tumour, tongue, sternum, heart, lung, liver, spleen, kidney, bladder, muscle, colon, ileum and stomach were weighed, and the amount of radioactivity was counted in a γ-well counter. In case of 1251 - 177Lu co - injection, dual-isotope counting was performed. For this purpose, crossover corrections from one radionuclide into the alternate window were made using a standard of each radionuclide. Radioactivity uptake was calculated as the percentage of the injected dose per gram of tissue (%ID/g), corrected for radioactive decay.

### RIT with ¹³¹I-L19-SIP

Three therapy experiments were performed using ¹³¹I-L19-SIP. The first therapy study was designed to assess the maximum tolerated dose (MTD) in FaDu bearing nude mice. The MTD, corresponding to a weight loss of 5 - 20%, was determined by monitoring the weight of FaDu bearing nude mice injected i.p. with diluent (0.9% Nacl) or increasing doses of ¹³¹I-L19-SIP. Groups of 5 mice containing 8 to 10 tumors with a mean volume of 148 ± 61 mm3 were treated with 37, 56 or 74 MBq ¹³¹I-L19-SIP (134 - 146 µg). In the second experiment FaDu bearing nude mice were used for studying the therapeutic efficacy of RIT alone and RIT in combination with Cetuximab. Mice bearing 1 or 2 subcutaneous FaDu xenografts were treated at day 0 with a single i.p. injection of ¹³¹I-L19-SIP (74 MBq, 110 µg) or Cetuximab (1 mg given 2 times a week i.p. for four weeks), or a combination of both. The mean tumour volume at the start of the experiment was 143 ± 85 mm3. Treatment groups consisted 8 mice with a total of 12 to 16 tumours per group. The third experiment was conform the second, but in this experiment mice were bearing HNX-OE xenografts (172 ± 101 mm3). Treatment groups consisted of 8 mice with a total of 13 to 15 tumours per group.

During treatment tumours were measured twice weekly for a planned period of three months and tumour volumes relative to the volume at the start of treatment were calculated. This period was extended for HNX-OE bearing mice to four months. Toxicity was monitored by measurement of body weight twice weekly. Mice were sacrificed when one of the tumours exceeded 1000 mm³. Average tumour volumes of the various groups were graphically depicted as long as at least 50% of the mice in a group were still alive.

An anti - tumour effect was also expressed as tumour growth delay. The tumour growth delay factor (GDF) was defined as the difference between the median values of the time required by tumours of treated and control animals to double their volume, divided by the median value of the time required by the tumours of the control mice to double their volume (Braakhuis BJM, Van Dongen GAMS, Vermorken JB, Snow GB. Preclinical in vivo activity of 2',2'-difluorodeoxycytidine (Gemcitabine) against human head and neck cancer. Cancer Res 1991;51:211-4). Median instead of mean values were used due to the fact that sometimes tumours regressed completely. In addition, survival curves were constructed.

### Statistics

Differences in tissue uptake between co - injected conjugates were statistically analyzed for each time point with Excel 2000 software (Microsoft) using the Student t-test for paired data. Therapy experiments were analyzed using Excel 2000 software and SPSS 11.0 software (SPSS). Differences in average tumour volume between the various groups were statistically analyzed for each time point with the Student's t-test for unpaired data. Survival was calculated using Kaplan-Meier curves. Two-sided significance levels were calculated, and P < 0.05 was considered statistically significant.

### Results

### Immunohistochemistry

The HNSCC xenograft lines FaDu and HNX-OE both expressed the ED-B domain of fibronectin as well as EGFR. ED-B expression was highest in FaDu, while EGFR expression was similar for both lines.

### Radiolabeling

Labelling of L19-SIP with ¹²⁵I or ¹³¹I for bio - distribution and therapy experiments resulted in overall labelling yields of > 70%. The radiochemical purity always exceeded 99%. Phosphor imager analysis of SDS-PAGE gels as well as HPLC analysis revealed optimal integrity of L19-SIP, irrespective whether the MAb was labelled with a low or a high dose of 131I. The immuno - reactivity of iodinated L19-SIP was always > 70% at the highest ED-B-resin concentration and > 95% at infinite antigen excess. Labelling of L19-SIP-p-SCN-Bz-DOTA with ¹⁷⁷Lu resulted in an overall labelling yield of 66%, while the results of quality tests were comparable to those obtained with iodinated L19-SIP. The specific activities of the conjugates prepared for the bio - distribution studies were 56 - 62 MBq/mg for iodinated L19-SIP and 73 MBq/mg for ¹⁷⁷Lu-L19-SIP, The specific activities for the therapy studies with ¹³¹I-L19-SIP studies were 623 - 789 MBq/mg.

### Biodistribution of L19-SIP

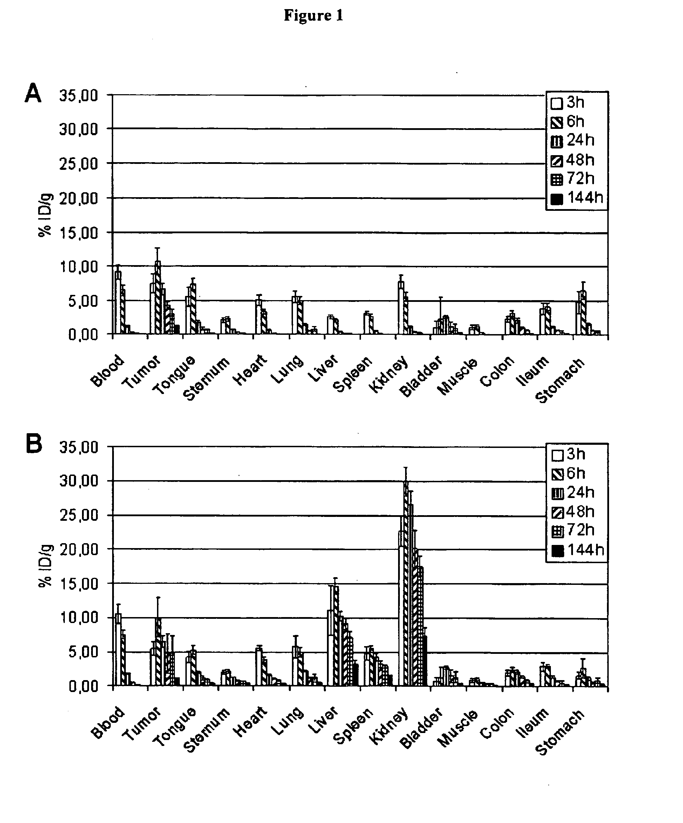

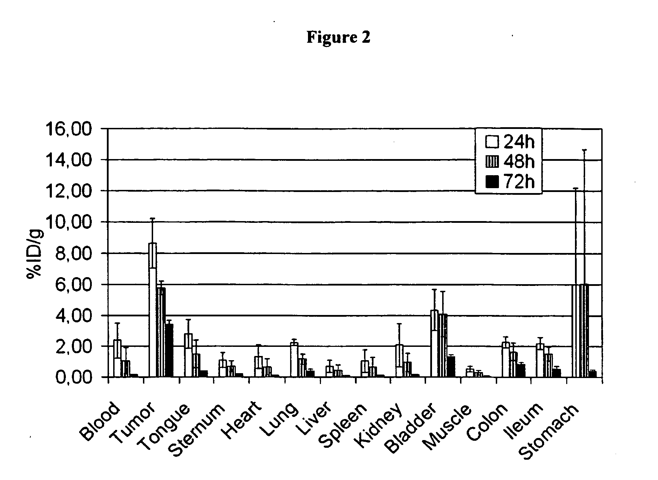

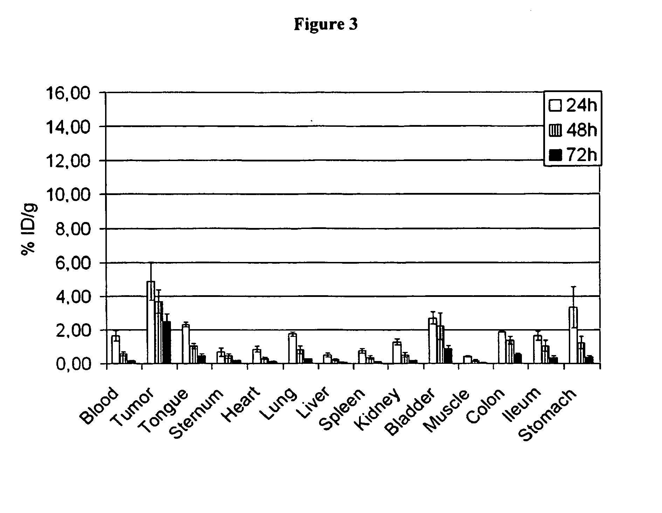

For comparison of the bio -distribution of ¹²⁵I-L19-SIP (non-residualizing radionuclide) with ¹⁷⁷Lu-L19-SIP (residualizing radionuclide) in tumour-bearing nude mice, both conjugates were co - injected. The average uptake in blood, tumour and normal tissues is shown by Fig. 1. Uptake of both radionuclides showed relatively small differences in blood, tumour, and most of the normal organs like tongue, sternum, heart, lung, bladder, muscle, colon and ileum. Large differences, however, were observed in kidney, liver, and spleen, with much higher uptake of ¹⁷⁷Lu than of ¹²⁵I. On the basis of these results, we concluded that non-residualizing radiolabels are better suited for RIT with L19-SIP than residualizing labels. On the basis of these results we decided to select ¹³¹I for RIT experiments with FaDu and HNX-OE bearing nude mice. Before starting these experiments, tumour targeting with ¹³¹I-L19-SIP was first compared in these two xenograft models. Uptake of ¹³¹I-L19-SIP in FaDu tumours was higher than in HNX-OE tumours, with uptake values at 24, 48 and 74 h. p.i. of 8.6 ± 1.6, 5.8 ± 0.4 and 3.4 ± 0.2 %ID/g, respectively, for FaDu, and 4.9 ± 1.1, 3.7 ± 0.7 and 2.5 ± 0.5 %ID/g, respectively, for HNX-OE (Fig. 2 and 3). Also tumour to non - tumour ratios were in general higher for the FaDu xenograft line (Table 3). While for example the tumour to blood ratios gradually increased for the FaDu xenograft line from 4.4 ± 1.8 at 24 h to 21.4 ± 1.7 at 72 h, for the HNX-OE xenograft line these values were 3.1 ± 1.2 and 15.1 ± 1.5, respectively.

Therapy of L19-SIP in FaDu and HNX-OE bearing nude mice

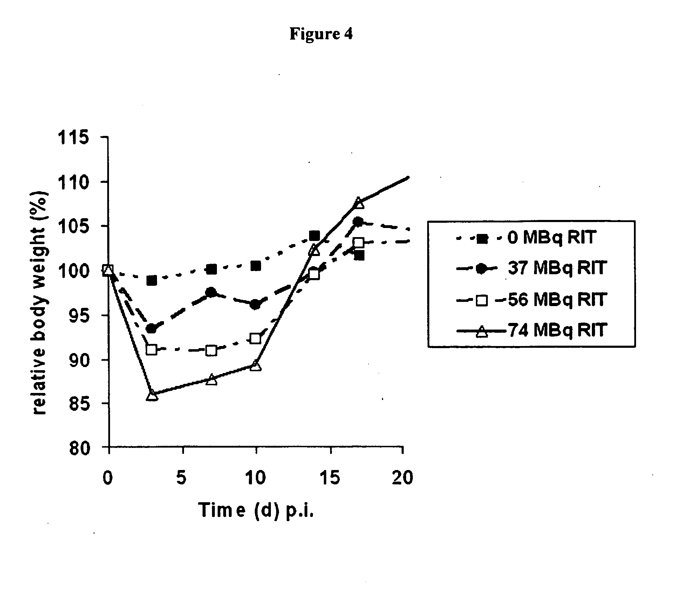

The first therapy study was designed to assess the maximum tolerated dose (MTD) of ¹³¹I-L19-SIP. The MTD was determined by monitoring the weight of FaDu bearing nude mice injected with diluent or 37, 56 or 74 MBq of 131I-L19-SIP. A dose dependent weight loss was observed and the MTD was established at 74 MBq (Fig 4). At this dose level weight recovered within 14 days, and no treatment related deaths were observed.

**TABLE 3:**

| **Timepoints** | **24 h** | | **48 h** | | **72 h** | |
|---|---|---|---|---|---|---|
| **Xenograft** | **FaDu** | **HNX-OE** | **FaDu** | **HNX-OE** | **FaDu** | **HNX-OE** |
| Tumor / Tissue ratios | | | | | | |
| T/Blood | 4.4 (1.8) | 3.1 (1.2) | 7.4 (3.7) | 6.3 (1.6) | 21.4 (1.7) | 15.1 (1.5) |
| T/Tongue | 3.4 (1.2) | 2.1 (0.6) | 4.7 (1.7) | 3.6 (1.1) | 9.3 (0.9) | 5.5 (1.6) |
| T/Sternum | 9.1 (3.7) | 8.2 (4.5) | 9.2 (3.2) | 8.3 (1.5) | 16.7 (1.3) | 14.1 (3.1) |
| T/Heart | 8.1 (3.6) | 6.0 (2.4) | 12.6 (6.7) | 11.5 (1.7) | 31.0 (3.2) | 22.7 (3.7) |
| T/Lung | 3.4 (1.7) | 2.8 (0.6) | 5.0 (1.3) | 4.7 (0.9) | 10.1 (3.5) | 9.9 (2.1) |
| T/Liver | 14.9 (6.8) | 10.7 (4.5) | 20.0 (10.3) | 18.1 (4.8) | 50.6 (5.8) | 38.4 (7.9) |
| T/Spleen | 11.3 (5.7) | 6.6 (2.0) | 14.2 (7.5) | 10.8 (1.8) | 31.4 (1.2) | 22.0 (5.1) |
| T/Kidney | 5.6 (2.7) | 3.9 (1.2) | 7.5 (2.9) | 7.2 (0.7) | 20.2 (3.3) | 14.2 (2.5) |
| T/Bladder | 2.3 (1.1) | 1.8 (0.2) | 1.6 (0.5) | 1.6 (0.5) | 2.8 (0.5) | 2.8 (0.5) |
| T/Muscle | 17.9 (6.4) | 11.9 (3.7) | 23.3 (9.7) | 22.4 (3.1) | 68.1 (15.5) | 47.5 (2.5) |
| T/Colon | 4.0 (0.9) | 2.6 (0.6) | 3.9 (1.2) | 2.7 (0.2) | 4.4 (0.9) | 4.6 (0.3) |
| T/Ileum | 4.2 (1.3) | 2.9 (0.6) | 4.1 (1.2) | 3.6 (0.4) | 8.5 (5.4) | 7.1 (1.8) |
| T/Stomach | 2.6 (1.5) | 1.7 (1.1) | 2.9 (2.2) | 3.1 (1.0) | 8.8 (1.8) | 6.3 (1.7) |

The therapy experiments were set up to compare the efficacy of RIT with ¹³¹1-L19-SIP in FaDu and HNX-OE bearing nude mice, either alone or in combination with twice-weekly injections of Cetuximab for four weeks.

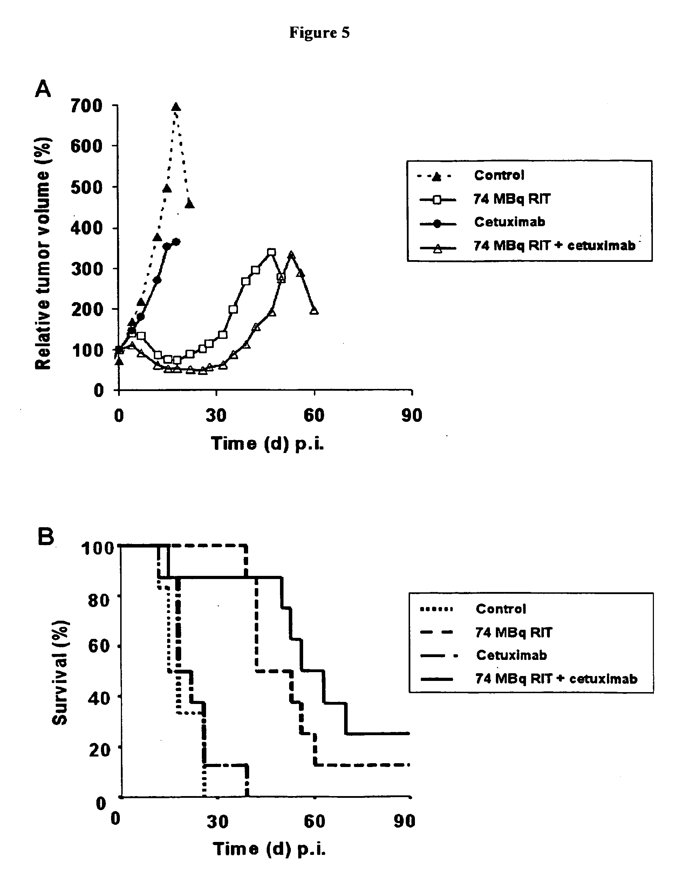

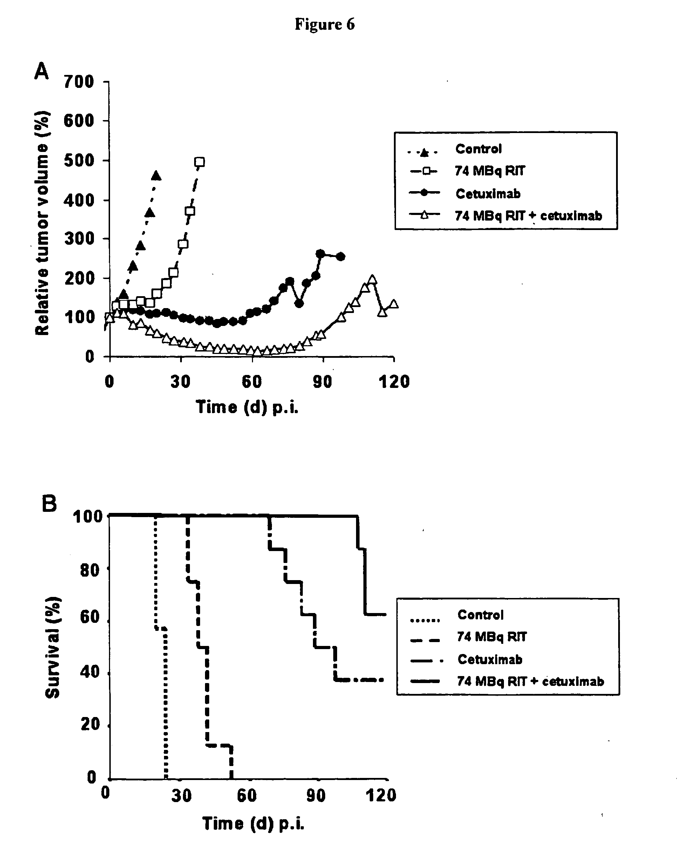

FaDu tumours in the control group showed exponential growth with a mean tumour volume doubling time of 6 days (Fig. 6A). Treatment with 74 MBq 131I-L19-SIP, caused size reduction of all tumours with a maximum decrease of average tumor volume to 74 ± 38% at day 18. At day 26, the tumours of this group had regrown to their initial volume. Thereafter tumours grew more or less exponentially. The combination of RIT (74 MBq) with Cetuximab showed an enhanced efficacy in comparison to RIT or Cetuximab treatment alone, while toxicity was not increased. Cetuximab treatment alone just slightly reduced the growth rate of FaDu tumours. When added to RIT, however, size reduction started earlier (day 5), reduction was more (50 ± 48% at day 26), while regrowth to the initial volume was later (day 35). The mean relative tumuor volume in the RIT + Cetuximab treatment group was significant smaller than in the RIT alone group from day 4 to day 42. GDF values for the different treatment groups were as follows: 74 MBq RIT: 4.8, Cetuximab: 0.2; and 74 MBq RIT + Cetuximab: 7.3. At the end of the observation period (day 90), 1 out of 8 mice was still alive in the 74 MBq RIT group, while for the 74 MBq RIT + Cetuximab group this was 2 out of 8 (Fig. 5B).

RIT with a single injection of ¹³¹I-L19-SIP was also evaluated in the second xenograft line HNX-OE (Fig. 6). The mean control tumour volume doubling time of this line was 8 days. In comparison to FaDu, the HNX-OE xenograft line was less sensitive for RIT (no reduction of the mean tumour size) and more sensitive for Cetuximab treatment. Also in this xenograft line, combined RIT + Cetuximab treatment showed the best therapeutic results without causing increased toxicity. Immediately after start of combination treatment, the size of all tumours decreased, with a maximum decrease to 14 ± 10% at day 62, while at the end of the observation period just 3 out of 14 tumours had a size larger than their initial volume. Consequently, all mice were still alive at day 90. In contrast, Cetuximab treatment alone resulted just in a minor reduction of the mean tumour size for a short duration, while just 50% of the mice were still alive at day 90. GDF values for the different treatment groups were as follows: 74 MBq RIT: 2.0, Cetuximab: 8.5; and 74 MBq RIT + Cetuximab: 12.8.

Since not all tumours had disappeared in the combination treatment group, the observation period was extended with 30 days. Survival after 120 days p.i. was 3 out of 8 for the Cetuximab alone group and 5 out of 8 for the RIT + Cetuximab treatment group.

### Discussion

Two radionuclides were evaluated as candidate for RIT with L19-SIP, the non-residualizing radionuclide ¹³¹I, and the residualizing radionuclide ¹⁷⁷Lu. If a MAb becomes internalized after binding to the tumour, the use of residualizing radionuclides for RIT might be advantageous due to higher tumor-to-nontumour ratios (23). Bio - distribution of co - injected ¹²⁵I-L19-SIP (125I as substitute for 131I to facilitate counting) and ¹⁷⁷Lu-L19-SIP in tumour bearing nude mice, showed just minor differences in uptake for tumour, blood, and most normal organs. In contrast, much higher uptake of ¹⁷⁷Lu than of ¹²⁵I was found in organs of conjugate catabolism kidney, liver, and spleen. On the basis of these results we decided to pursue with ¹³¹I for RIT, and not with a residualizing radionuclide. While tumour uptake was equal with both radionuclides, tumor-to-nontumor ratios were lower for the residualizing 111 In label. Dosimetric calculation using 125I and ¹¹¹In as surrogates for the therapeutic radionuclides ¹³¹I and ⁹⁰Y, revealed the most favourable therapeutic index for ¹³¹I-L19-SIP.

The MTD for single i.p. administration of ¹³¹I-L19-SIP was assessed at 74 MBq in FaDu-bearing mice. In our MTD study, weight loss was used as gross toxicity criterion. Berndorff et al. (Berndorff D, Borkowski S, Sieger S, et al. Radioimmunotherapy of solid tumors by targeting extra domain B fibronectin: Identification of the best-suited radioimmunoconjugate. Clin Cancer Res 2005; 1 1:7053s-63s) found the same MTD value for 131I-L19-SIP, but they arrived at this value by a totally different approach.

RIT with ¹³¹I-L19-SIP caused a significant tumour growth delay and improved survival in both HNSCC xenograft lines. However, no cures were observed. Anti-tumour effects were more pronounced for the FaDu than for the HNX-OE line, which might be related to the higher ED-B expression in this xenograft line, and the accompanying higher tumour uptake of L19-SIP as observed in bio - distribution experiments (Fig 2 and 3). Treatment of FaDu bearing nude mice with 74 MBq ¹³¹I-L19-SIP caused size reduction of all tumours, while at day 26 the tumours had regrown to their initial volume. Anti-tumour effects in this human HNSCC xenograft line were more pronounced than previously observed for mouse embryonal teratocarcinoma F9 xenografts (8). While L19-SIP showed very high tumour uptake in the F9 model, with tumour uptake values almost twice as high as observed for FaDu (i.e. 17.5, 16.7, 15.3 and 12.0 %ID/g at 3, 6, 24 and 48 h p.i.), tumour growth was delayed for just about 10 days. Since murine F9 teratocarcinoma is a very aggressive tumour (doubling time - 48 hours), it might be that the potential of ¹³¹I-L19-SIP became underestimated in these previous RIT studies.

Several options can be explored to optimize the efficacy of RIT with ¹³¹I-L19-SIP, like application of RIT dose fractionation or repeated injections, or combination with chemotherapy (DeNardo GL, Schlom J, Buchsbaum DJ, et al. Rationales, evidence, and design considerations for fractionated radioimmunotherapy. Cancer 2002;94:1332-48; Blumen-thal RD, Alisauskas R, Juweid M, Sharkey RM, Goldenberg DM. Defining the optimal spacing between repeat radioantibody doses in experimental models. Cancer 1997;80:2624-35; DeNardo SJ, Kukis DL, Kroger LA, et al. Synergy oftaxol and radioimmunotherapy with yttrium-90-labeled chimeric L6 antibody: efficacy and toxicity in breast cancer xenografts. Proc Natl Acad Sci USA 1997;94:4000-4). Alternatively, RIT with ¹³¹I-L19-SIP can be used in combination treatment enabling simultaneous targeting of multiple pathways. In HNSCC, angiogenesis has been linked with tumour progression and worse outcome and it might represent a resistance mechanism to anti-EGFR agents (Caponigro F, Formato R, Caraglia M, Normanno N, Iaffaioli RV. Monoclonal antibodies targeting epidermal growth factor receptor and vascular endothelial growth factor with focus on head and neck tumors. Curr Opin Oncol 2005; 17:212-7; Van Cruijsen H, Giaccone G, Hoekman K. Epidermal growth factor receptor and angiogenesis: Opportunities for combined anticancer strategies. Int J Cancer 2005;117:883-8). In this context it was interesting to combine ¹³¹I-L19-SIP with Cetuximab treatment. Cetuximab was a logical anti-EGFR candidate as this MAb had shown improved locoregional control and survival in combination with radiotherapy in a phase III study in locally advanced inoperable HNSCC (Bonner JA, Giralt J, Harari P, et al. Cetuximab prolongs survival in patients with locally advanced squamous cell carcinoma of head and neck: a phase III study of high dose radiation therapy with or without cetuximab. J Clin Oncol, ASCO Annual meeting Proceedings (Post Meeting Edition) 2004;22(No 14S), Abstr 5507). Our results showed an enhanced efficacy when RIT with 131I-L19-SIP was combined with Cetuximab treatment. While the FaDu xenograft line was relatively sensitive for RIT and xenograft line HNX-OE for Cetuximab, best growth delay, survival and cure rates were observed for both lines when treatment modalities were combined. Radiation response improvement by anti-EGFR treatment was also observed in previous RIT studies in HNSCC-bearing nude mice (Van Gog FB, Brakenhoff RH, Stigter-Van Walsum M, Snow GB, Van Dongen GAMS. Perspectives of combined radioimmunotherapy and anti-EGFR antibody therapy for the treatment of residual head and neck cancer. Int J Cancer 1998;77:13-8), as well as in clinical studies upon external beam irradiation of head and neck cancer (Harari PM. Promising new advances in head and neck radiotherapy. Ann Oncol 2005;16:vi13-vi19). An additional advantage for the combination with an anti-EGFR MAb, in comparison with other agents studied in combination with RIT, like cytostatic drugs, is the low toxicity of anti-EGFR MAbs. In the present study combination of RIT with Cetuximab treatment did not result in increased toxicity. Also in patients an increase of RIT-induced dose-limiting toxicity, which is mainly bone marrow toxicity, is not expected, in view of the fact that expression of EGFR is absent in the bone marrow.

## Claims

1. A combination comprising at least (i) a fusion protein and (ii) an anti-EGFR-antibody, wherein the fusion protein in its monomeric form comprises
an antibody - part, specifically recognising the ED-B - fibronectin domain and an εₛ₂-CH4 domain part
and
wherein the fusion protein is present in dimeric form
and
wherein the fusion protein is conjugated to a radioisotope.

2. A combination according to claim 1, wherein the antibody - part binds specifically to the ED-B oncofetal fibronectin domain with sub-nanomolar affinity.

3. A combination according to any of claims 1 to 2, wherein the antibody-part comprises the sequences according to SEQ ID no. 6 to 11.

4. A combination according to any of claims 1 to 3, wherein the antibody-part comprises one variable heavy (Vh) chain according to Seq. Id. No. 01 and one variable light (Vl) chain according to Seq. Id. No. 02.

5. A combination according to any of claims 1 to 4, wherein the εₛ₂-CH4 domain part comprises a sequence according to SEQ. ID. No. 04.

6. A combination according to any of claims 1 to 5, wherein an anti-EGFR-antibody is Cetuximab.

7. A combination according to any one of the proceeding claims, wherein the radioisotope is selected from ¹²³I, ¹²⁴I,¹²⁵I, ¹³¹I, ^{99m}Tc, ¹⁸⁶Re, ¹⁸⁸Re ²⁰³Pb, ⁶⁷Ga, ⁶⁸Ga, ⁴³Sc, ⁴⁷Sc, ^{110m}In, ¹¹¹In, ⁹⁷Ru, ⁶²Cu, ⁶⁴Cu, ⁶⁷Cu, ⁶⁸Cu, ⁸⁶Y, ⁹⁰Y ⁸⁸Y, ¹²¹Sn, ¹⁵³Sm, ¹⁶⁶Ho, ¹⁰⁵Rh, ¹⁷⁷Lu, ¹⁷²Lu, ²¹¹At and / or ¹⁸F or a mixture thereof.

8. A combination according to claim 7, wherein a ¹³¹I-labeled fusion protein is used, and wherein the fusion protein in its monomeric form contains a Vl domain of human recombinant antibody L19, a Vh domain of human recombinant antibody L19 and an εₛ₂-CH4 domain.

9. A combination according to claim 7 or 8, wherein the fusion protein is ¹³¹I labelled L19-small immunoprotein (L19-SIP).

10. A combination according to claim 9, wherein the combination comprises ¹³¹I labelled L 19-small immunoprotein and an anti-EGFR-antibody.

11. A combination according to any one of the claims 1 to 10, for use as a medicament.

12. A combination according to any one of claims 1 to 11, for use as a medicament for the treatment of cancer.

13. A combination according to claim 12, wherein the cancer is pancreatic cancer.

## Patentansprüche

1. Kombination, umfassend zumindest (i) ein Fusionsprotein und (ii) einen Anti-EGFR-Antikörper, wobei das Fusionsprotein in seiner monomeren Form Folgendes umfasst:
einen Antikörperteil, der spezifisch die Fibronektin-ED-B-Domäne erkennt, und
einen εₛ₂-CH4-Domänenteil
und
wobei das Fusionsprotein in dimerer Form vorliegt
und
wobei das Fusionsprotein mit einem Radioisotop konjugiert ist.

2. Kombination nach Anspruch 1, wobei der Antikörperteil mit subnanomolarer Affinität spezifisch an die onkofötale Fibronektin-ED-B-Domäne bindet.

3. Kombination nach einem der Ansprüche 1 bis 2, wobei der Antikörperteil die Sequenzen gemäß SEQ ID NO. 6 bis 11 umfasst.

4. Kombination nach einem der Ansprüche 1 bis 3, wobei der Antikörperteil eine variable schwere Kette (Vh) gemäß SEQ ID NO. 01 und eine variable leichte Kette (V1) gemäß SEQ ID NO. 02 umfasst.

5. Kombination nach einem der Ansprüche 1 bis 4, wobei der εₛ₂-CH4-Domänenteil eine Sequenz gemäß SEQ ID NO. 04 umfasst.

6. Kombination nach einem der Ansprüche 1 bis 5, wobei es sich bei dem Anti-EGFR-Antikörper um Cetuximab handelt.

7. Kombination nach einem der vorhergehenden Ansprüche, wobei das Radioisotop aus der Reihe ¹²³I, ¹²⁴I, ¹²⁵I, ¹³¹I, ^{99m}Tc, ¹⁸⁶Re, ¹⁸⁸Re, ²⁰³Pb, ⁶⁷Ga, ⁶⁸Ga, ⁴³Sc, ⁴⁷Sc, ^{110m}In, ¹¹¹In , ⁹⁷Ru, ⁶²Cu, ⁶⁴Cu, ⁶⁷Cu, ⁶⁸Cu, ⁸⁶Y, ⁹⁰Y, ⁸⁸Y, ¹²¹Sn, ¹⁵³Sm, ¹⁶⁶Ho, ¹⁰⁵Rh, ¹⁷⁷Lu, ¹⁷²Lu, ²¹¹At und/oder ¹⁸F oder einer Mischung davon stammt.

8. Kombination nach Anspruch 7, wobei ein ¹³¹I-markiertes Fusionsprotein verwendet wird, und wobei das Fusionsprotein in seiner monomeren Form eine Vl-Domäne des humanen rekombinanten Antikörpers L19, eine Vh-Domäne des humanen rekombinanten Antikörpers L19 und eine εₛ₂-CH4-Domäne umfasst.

9. Kombination nach Anspruch 7 oder 8, wobei es sich bei dem Fusionsprotein um das ¹³¹I-markierte kleine L19-Immunprotein (L19-SIP) handelt.

10. Kombination nach Anspruch 9, wobei die Kombination das ¹³¹I-markierte kleine L19-Immunprotein und einen Anti-EGFR-Antikörper umfasst.

11. Kombination nach einem der Ansprüche 1 bis 10 zur Verwendung als Arzneimittel.

12. Kombination nach einem der Ansprüche 1 bis 11 zur Verwendung als Arzneimittel für die Behandlung von Krebs.

13. Kombination nach Anspruch 12, wobei es sich bei dem Krebs um Pankreaskrebs handelt.

## Revendications

1. Combinaison comprenant au moins (i) une protéine de fusion et (ii) un anticorps anti-EGFR, dans laquelle la protéine de fusion, sous sa forme monomère, comprend une partie anticorps, qui d'une manière spécifique reconnaît le domaine ED-B de la fibronectine, et une partie domaine εₛ₂-CH4, et dans laquelle la protéine de fusion est présente sous forme dimère, et dans laquelle la protéine de fusion est conjuguée à un radio-isotope.

2. Combinaison selon la revendication 1, dans laquelle la partie anticorps se lie d'une manière spécifique au domaine ED-B de la fibronectine oncofoetale, avec une affinité sous-nanomolaire.

3. Combinaison selon l'une quelconque des revendications 1 à 2, dans laquelle la partie anticorps comprend les séquences selon SEQ ID N°6 à 11.

4. Combinaison selon l'une quelconque des revendications 1 à 3, dans laquelle la partie anticorps comprend une chaîne lourde variable (Vh) selon SEQ ID N°01 et une chaîne légère variable (Vl) selon SEQ ID N°02.

5. Combinaison selon l'une quelconque des revendications 1 à 4, dans laquelle la partie domaine εₛ₂-CH4 comprend une séquence selon SEQ ID N°04.

6. Combinaison selon l'une quelconque des revendications 1 à 5, dans laquelle un anticorps anti-EGFR est le cétuximab.

7. Combinaison selon l'une quelconque des revendications précédentes, dans laquelle le radio-isotope est choisi parmi ¹²³I, ¹²⁴I, ¹²⁵I, ¹³¹I, ^{99m}Tc, ¹⁸⁶Re, ¹⁸⁸Re, ²⁰³Pb , ⁶⁷Ga, ⁶⁸Ga, ⁴³Sc, ⁴⁷Sc, ^{110m}In, ¹¹¹In, ⁹⁷Ru, ⁶²Cu, ⁶⁴Cu, ⁶⁷Cu, ⁶⁸Cu, ⁸⁶Y, ⁹⁰Y, ⁸⁸Y, ¹²¹Sn, ¹⁵³Sm, ¹⁶⁶Ho, ¹⁰⁵Rh, ¹⁷⁷Lu, ¹⁷²Lu, ²¹¹At et/ou ¹⁸F, ou un mélange de ceux-ci.

8. Combinaison selon la revendication 7, dans laquelle on utilise une protéine de fusion marquée au ¹³¹I, et dans laquelle la protéine de fusion sous sa forme monomère contient un domaine Vl de l'anticorps recombinant humain L19, un domaine Vh de l'anticorps recombinant humain L19 et un domaine εₛ₂-CH4.

9. Combinaison selon la revendication 7 ou 8, dans laquelle la protéine de fusion est la petite immunoprotéine de L19 marquée au ¹³¹I (L19-SIP).

10. Combinaison selon la revendication 9, ladite combinaison comprenant une petite immunoprotéine de L19 marquée au ¹³¹I et un anticorps anti-EGFR.

11. Combinaison selon l'une quelconque des revendications 1 à 10, pour utilisation en tant que médicament.

12. Combinaison selon l'une quelconque des revendications 1 à 11, pour utilisation en tant que médicament pour le traitement du cancer.

13. Combinaison selon la revendication 12, dans laquelle le cancer est le cancer du pancréas.
